# EUROPEAN PATENT APPLICATION

(11) **EP 3 231 352 A1**
(43) Date of publication of application: **18.10.2017**
(21) Application number: 15868596.6
(22) Date of filing: 21.10.2015
(51) Int. Cl.: A61B 1/06, G02B 23/26

(54) **ILLUMINATION DEVICE, METHOD FOR CONTROLLING ILLUMINATION DEVICE, AND IMAGE-ACQUIRING SYSTEM**

(30) Priority: 09.12.2014 JP 2014248797
(71) Applicant: Sony Corporation, Tokyo 108-0075 (JP)
(72) Inventor: OKI, Tomoyuki, Tokyo 108-0075 (JP)
(74) Representative: D Young & Co LLP
(86) International application number: PCT/JP2015/079732
(87) International publication number: WO 2016/092958

(57) **Abstract**

[Object] Proposed is a new and improved illuminating device, a control method for the illuminating device, and an image acquisition system which can adjust light amounts of a plurality of respective light sources on the basis of correlations between a luminance acquired from a light receiving unit and the light amounts of the emission light beams so as to precisely adjust the color temperature in the light receiving unit.

[Solution] An illuminating device includes: a white light source configured to emit white light; RGB light sources light amounts of respective emission light beams of which are independently adjustable; a multiplexing unit configured to multiplex the white light and the emission light beams into illumination light; and a control unit configured to control the light amounts of the respective emission light beams.

## Description

### Technical Field

The present disclosure relates to an illuminating device, a control method for an illuminating device, and an image acquisition system.

### Background Art

Conventionally, there has been proposed a technique that uses RGB light sources instead of a lamp light source such as a xenon lamp or a Halogen lamp in an image acquisition system such as an endoscopic system and an electronic microscope system. A lamp light source, which is incapable of electrically adjusting the light amounts of the emission light beams, has to constantly keep illuminating with a maximum power, whereas the RGB light sources allow for electrically controlling the light amounts of emission light beams having respective colors. Therefore, the RGB light sources allow for adjusting the color temperature of the illumination light by controlling the light amounts of emission light beams having respective colors of R, G, and B. For example, Patent Literature 1 discloses a light source device having visible light LEDs with stand-alone RGB light sources as a photographing system available for an endoscopic system or an electronic microscope system.

In Patent Literature 1, the intensity of emission light beams having respective colors of R, G, and B is adjusted with the visible light LED for all R, G, and B turned on, thereby adjusting the white balance of outputs from an image sensor. Specifically, Patent Literature 1 describes that the intensity of emission light beams having respective colors of R, G, and B is adjusted on the basis of a color temperature detected by a white balance adjustment device on the basis of the outputs from the image sensor in order to adjust the white balance. In addition, Patent Literature 1 describes that the intensity of emission light beams having respective colors of R, G, and B is adjusted according to an input by a user looking at monitor display based on the outputs from the image sensor in order to adjust the white balance.

### Citation List

### Patent Literature

- Patent Literature 1:: JP 2012-29728A

### Disclosure of Invention

### Technical Problem

In an illuminating device which requires such a color temperature adjustment of the illumination light, a desired color temperature, light amount, or the like of the illumination light varies depending on the imaging object, the purpose of imaging, or the user's preference. In such an illuminating device, therefore, it is desired to further improve the degree of freedom for adjusting color temperature and light amount.

Therefore, the presents disclosure proposes a new and improved illuminating device, a control method for the illuminating device, and an image acquisition system that allow for improving the degree of freedom for adjusting color temperature and light amount by multiplexing white light emitted from a white light source and emission light beams of RGB light sources into illumination light, while adjusting at least the light amounts of the emission light beams having respective colors of R, G, and B emitted from the RGB light sources.

### Solution to Problem

According to the present disclosure, there is provided an illuminating device including: a white light source configured to emit white light; RGB light sources light amounts of respective emission light beams of which are independently adjustable; a multiplexing unit configured to multiplex the white light and the emission light beams into illumination light; and a control unit configured to control the light amounts of the respective emission light beams.

Further, according to the present disclosure, there is provided a control method for an illuminating device, the control method including: controlling light amounts of respective emission light beams emitted from RGB light sources so as to acquire illumination light of a desired luminance, the respective emission light beams being multiplexed with white light emitted from a white light source.

Further, according to the present disclosure, there is provided an image acquisition system including: a white light source configured to emit white light; RGB light sources light amounts of respective emission light beams of which are independently adjustable; a multiplexing unit configured to multiplex the white light and the emission light beams into illumination light; a light receiving unit configured to receive the illumination light; and a control unit configured to control light amounts of the respective emission light beams.

### Advantageous Effects of Invention

According to the present disclosure as described above, it becomes possible to improve the degree of freedom for adjusting color temperature and light amount by multiplexing white light emitted from a white light source and emission light beams of RGB light sources into illumination light, while adjusting at least the light amounts of the emission light beams having respective colors of R, G, and B emitted from the RGB light sources. Note that the effects described above are not necessarily limitative. With or in the place of the above effects, there may be achieved any one of the effects described in this specification or other effects that may be grasped from this specification.

### Brief Description of Drawings

[FIG. 1] FIG. 1 is a block diagram illustrating an image acquisition system according to a first embodiment of the present disclosure.
[FIG. 2] FIG. 2 is a schematic diagram illustrating an example of a configuration of a green light source according to the embodiment.
[FIG. 3] FIG. 3 is a schematic diagram illustrating an RGB multiplexing module including a light monitor unit according to the embodiment.
[FIG. 4] FIG. 4 is a flowchart illustrating an example of white balance adjustment processing according to the embodiment.
[FIG. 5] FIG. 5 is a flowchart illustrating an example of correlation acquisition processing according to the embodiment.
[FIG. 6] FIG. 6 illustrates a correlation between a light monitor value of a red light source and a luminance value in a light receiving unit.
[FIG. 7] FIG. 7 illustrates a correlation between a light monitor value of a green light source and a luminance value in a light receiving unit.
[FIG. 8] FIG. 8 illustrates a correlation between a light monitor value of a blue light source and a luminance value in a light receiving unit.
[FIG. 9] FIG. 9 is a block diagram illustrating an image acquisition system according to a second embodiment of the present disclosure.
[FIG. 10] FIG. 10 is a block diagram illustrating an image acquisition system according to a third embodiment of the present disclosure.
[FIG. 11] FIG. 11 is a schematic diagram illustrating a multiplexing module including a light monitor unit according to the embodiment.
[FIG. 12] FIG. 12 is a flowchart illustrating an example of white balance adjustment processing according to the embodiment.
[FIG. 13] FIG. 13 is a flowchart illustrating an example of correlation acquisition processing according to the embodiment.
[FIG. 14] FIG. 14 illustrates a correlation between a light monitor value of a white light source and a luminance value of red light in a light receiving unit.
[FIG. 15] [FIG. 15 illustrates a correlation between a light monitor value of a white light source and a luminance value of green light in a light receiving unit.
[FIG. 16] FIG. 16 illustrates a correlation between a light monitor value of a white light source and a luminance value of blue light in a light receiving unit.
[FIG. 17] FIG. 17 is a flowchart illustrating an example of color temperature adjustment processing.
[FIG. 18] FIG. 18 is a flowchart illustrating an example of light amount adjustment processing.
[FIG. 19] FIG. 19 is a flowchart illustrating an example of multiplexing ratio adjustment processing.
[FIG. 20] FIG. 20 is a flowchart illustrating an example of deterioration determination processing of a light source.
[FIG. 21] FIG. 21 is a block diagram illustrating an image acquisition system according to a fifth embodiment of the present disclosure.
[FIG. 22] FIG. 22 is a schematic diagram illustrating a multiplexing module having a light monitor unit (color sensor) according to the embodiment.
[FIG. 23] FIG. 23 is a schematic diagram illustrating a configuration example of a color sensor.
[FIG. 24] FIG. 24 is an explanatory diagram illustrating an example of spectral sensitivity property of a color sensor.

### Mode(s) for Carrying Out the Invention

Hereinafter, (a) preferred embodiment(s) of the present disclosure will be described in detail with reference to the appended drawings. In this specification and the appended drawings, structural elements that have substantially the same function and structure are denoted with the same reference numerals, and repeated explanation of these structural elements is omitted.

Note that a description will be provided in the following order.
<1. First embodiment (example of controlling light amounts of emission light beams having respective colors of R, G, and B)>
   [1.1. Overall configuration example of image acquisition system]
      (1.1.1. Configuration example of illuminating device)
      (1.1.2. Configuration example of imaging processing device)
   [1.2. Control processing example of illuminating device]
<2. Second embodiment (example having a common control unit)>
<3. Third embodiment (example of controlling light amounts of white light and emission light beams having respective colors of R, G, and B)
   [3.1. Overall configuration example of image acquisition system]
   [3.2. Control processing example of illuminating device]
      (3.2.1. White balance adjustment processing example)
      (3.2.2. Color temperature adjustment processing example)
      (3.2.3. Light amount adjustment processing example)
      (3.2.4. Multiplexing ratio adjustment processing example)
<4. Fourth embodiment (example in which deterioration determination processing function of light source is provided)>
<5. Fifth embodiment (example of using color sensor)>

Hereafter, it is assumed in the present specification that "white light" refers to light emitted from a white light source, "emission light beams" refers to light emitted from each of RGB light sources, and "illumination light" refers to light radiated from an illuminating device.

### <1. First embodiment>

### [1.1. Overall configuration example of image acquisition system]

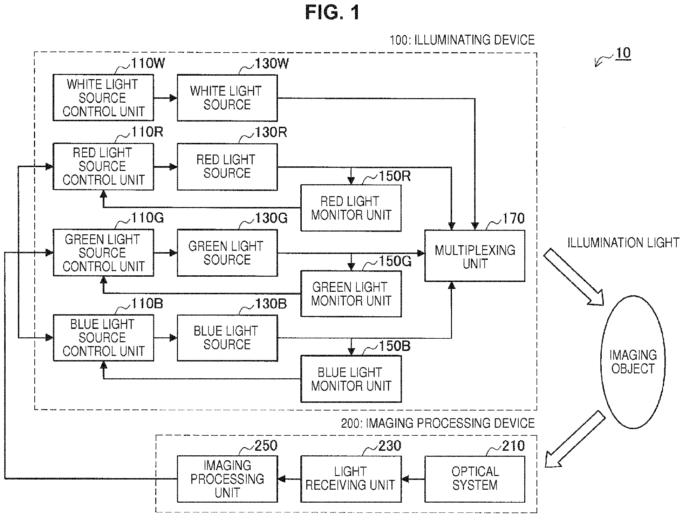

First of all, the schematic configuration of an image acquisition system 10 according to a first embodiment of the present disclosure will be described with reference to FIG. 1. FIG. 1 is a block diagram illustrating the overall configuration of the image acquisition system 10 according to the present embodiment. This image acquisition system 10 includes an illuminating device 100 and an imaging processing device 200, and is configured, for example, as an endoscopic system. However, the endoscopic system is an example of the image acquisition system 10, so that the image acquisition system 10 may be another system such as an electronic microscope system.

### (1.1.1. Configuration example of illuminating device)

The illuminating device 100 includes a white light source 130W, a red light source 130R, a green light source 130G, a blue light source 130B, a white light source control unit 110W, a red light source control unit 110R, a green light source control unit 110G, a blue light source control unit 110B, and a multiplexing unit 170. The illuminating device 100 further includes a red light monitor unit 150R, a green light monitor unit 150G, and a blue light monitor unit 150B.

### (White light source)

The white light source 130W includes, for example, a blue LED (Light Emitting Diode), and a fluorescent body which is excited by emitted light from the blue LED to emit yellow light. Unlike a lamp light source such as a xenon lamp or a Halogen lamp, the white light source 130W allows for electrically adjusting the light amount of emitted white light. However, the white light source 130W is not limited to the aforementioned example and may be of any type that allows for emitting white light.

### (Light sources for R, G, and B)

The red light source 130R includes a semiconductor laser such as a Gain quantum well structure laser diode, and a blue light source 130B includes a semiconductor laser such as a GaInN quantum well structure laser diode. The green light source 130G includes a solid-state laser that is excited, for example, by a semiconductor laser. In the illuminating device 100 according to the present embodiment, the RGB light sources include three-color light sources controlled by the semiconductor laser, and unlike a lamp light source such as a xenon lamp or a Halogen lamp, it is possible to electrically adjust the light amounts of the emission light beams.

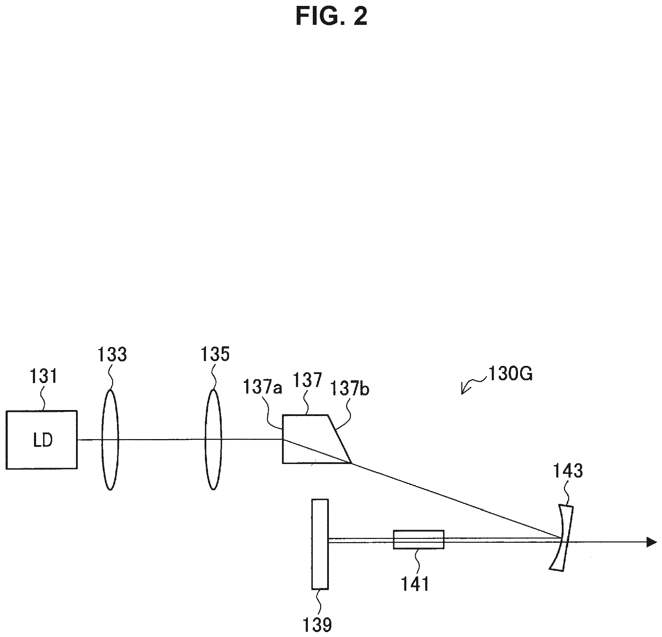

FIG. 2 is a schematic diagram illustrating a configuration example of the green light source 130G including a solid-state laser. The green light source 130G exemplified in FIG. 2 includes an excitation light source 131 including an AlGaAs quantum well structure laser diode, a condensing lenses 133 and 135, and an optical crystal 137 including YVO₄. Further, the green light source 130G includes a resonator mirror 139, a wavelength conversion element 141 made of PPMgSLT, and a reflection unit 143 made of a concave mirror. The condensing lenses 133 and 135 and the optical crystal 137 are disposed in this order on the light path of light emitted from the excitation light source 131.

One end face of the optical crystal 137 on the side of the excitation light source 131 is configured to be a vertical face perpendicular to the optical axis and configured to be a resonator mirror having a high reflection film 137a. Further, the other end face of the optical crystal 137 is configured to be a slanted face having an angle except the Brewster's angle, and this slanted face is provided with an anti-reflection film 137b. The reflection unit 143 is disposed on the emission light path of light emitted from the optical crystal 137. The wavelength conversion element 141 is disposed on the light path of the light reflected by the reflection unit 143. The wavelength conversion element 141 is provided with anti-reflection films on both faces thereof. The resonator mirror 139 is provided on the opposite side of the reflection unit 143 across the wavelength conversion element 141.

In this green light source 130G, the excitation light emitted from the excitation light source 131 is configured to be a basic wave having a beam shape through the condensing lenses 133 and 135 to enter the optical crystal 137. The optical crystal 137 is excited by the incident light to emit new laser light. The emitted light is reflected by the reflection unit 143 to be radiated to the wavelength conversion element 141, transmitted through the wavelength conversion element 141, and reflected by the resonator mirror 139. The light emitted from the wavelength conversion element 141 is a conversion wave and the conversion wave is emitted as emission light beams after having transmitted through the reflection unit 143.

Note that the aforementioned semiconductor laser or solid-state laser is an example of the red light source 130R, the green light source 130G, and the blue light source 130B, and light sources of other types may be used. The green light source 130G may include a semiconductor laser. In addition, the light source is not limited to the light sources for three colors R, G, and B but may be the light sources for four colors or the like, and the number of light sources is not limited. However, if it is the laser light source, it causes less diffusion of the emission light beams and makes it easier for the light monitor unit to detect the light amount.

### (Light monitor units)

The red light monitor unit 150R, the green light monitor unit 150G, and the blue light monitor unit 150B are configured with photodiodes, for example. The red light monitor unit 150R detects the light amount of the emission light beams from the red light source 130R. The green light monitor unit 150G detects the light amount of the emission light beams from the green light source 130G. The blue light monitor unit 150B detects the light amount of the emission light beams from the blue light source 130B. Explaining the red light monitor unit 150R, the red light monitor unit 150R configured with the photodiode receives a part of the emission light beams (red light) emitted from the red light source 130R, converts the light amount of the received light into a voltage signal, and transmits the voltage signal to the red light source control unit 110R. Similarly, the green light monitor unit 150G and the blue light monitor unit 150B receive parts of green light and blue light, convert the light amounts of the received light beams into voltage signals, and transmit the voltage signals to the green light source control unit 110G and the blue light source control unit 110B, respectively.

### (Multiplexing unit)

The multiplexing unit 170 multiplexes the white light, the red light, the green light, and the blue light emitted from the white light source 130W, the red light source 130R, the green light source 130G, and the blue light source 130B, respectively. The illuminating device 100 according to the present embodiment changes the balance of the luminances Lr, Lg, and Lb of the red light, the green light, and the blue light by adjusting the light amounts of the red light, the green light, and the blue light, respectively. Accordingly, color temperature of the illumination light after having been multiplexed with the white light is adjusted.

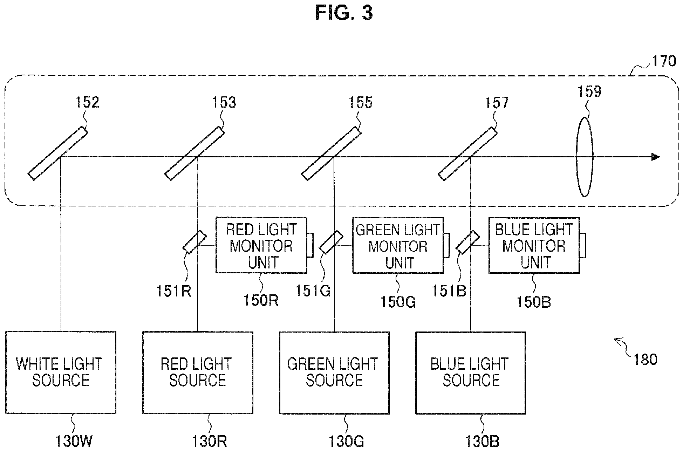

FIG. 3 is a schematic diagram illustrating a configuration example of a multiplexing module 180 with a light monitor, including the multiplexing unit 170. In the multiplexing module 180, the multiplexing unit 170 has a mirror 152 and dichroic mirrors 153, 155, and 157. The dichroic mirrors 153, 155, and 157 respectively reflect light of particular wavelengths. The dichroic mirrors 153, 155, and 157, on the other hand, transmit light of other wavelengths. In the example of FIG. 3, the white light emitted from the white light source 130W is reflected by the mirror 152 and changes its course toward a lens 159. The mirror 152 may be a dichroic mirror.

The red light emitted from the red light source 130R is reflected by the dichroic mirror 153 and changes its course toward the lens 159. On this occasion, the white light sent from the mirror 152 directly transmits through the dichroic mirror 153. In addition, the green light emitted from the green light source 130G is reflected by the dichroic mirror 155 and changes its course toward the lens 159. On this occasion, the white light and the red light sent from the mirror 153 directly transmit through the dichroic mirror 155. Furthermore, the blue light emitted from the blue light source 130B is reflected by the dichroic mirror 157 and changes its course toward the lens 159. On this occasion, the white light, the red light, and the green light sent from the dichroic mirror 155 directly transmit through the dichroic mirror 157.

As thus described, the white light and light having respective colors of R, G, and B are directed along the same optical axis to be superimposed. In the example of the multiplexing module 180, after the red light having the longest wavelength is multiplexed with the white light, the green light having the next longest wavelength is multiplexed thereon, and further the blue light having the shortest wavelength is multiplexed thereon. The multiplexed light is further collected by the lens 159 to be emitted as illumination light. In the case of the endoscopic system according to the present embodiment, the emitted illumination light is directed to the tip of the endoscopic probe and radiated therefrom to illuminate the target portion.

In the multiplexing module 180, a part of the red light emitted from the red light source 130R is input into the red light monitor unit 150R by the use of a light sampler 151R before the multiplexing. Thereby, the light amount of the red light can be detected. Similarly, the parts of the green light and the blue light emitted from the green light source 130G and the blue light source 130B are input into the green light monitor unit 150G and the blue light monitor unit 150B by the use of light samplers 151G and 151B, respectively, and the light amounts can be detected.

### (Light source control unit)

The white light source control unit 110W illustrated in FIG. 1 drives and controls the white light source 130W, and the red light source control unit 110R drives and controls the red light source 130R. In addition, the green light source control unit 110G drives and controls the green light source 130G, and the blue light source control unit 110B drives and controls the blue light source 130B. Among these, the white light source control unit 110W controls the drive current supplied to the white light source 130W. In addition, the red light source control unit 110R controls the drive current supplied to the red light source 130R, on the basis of the correlation between a luminance Lr detected by a light receiving unit 230 of an imaging processing device 200 and a light monitor value Qr detected by a red light monitor unit 150R. The green light source control unit 110G or the blue light source control unit 110B similarly controls the drive current of the green light source 130G or the blue light source 130B, on the basis of the correlation between the luminance Lg, Lb detected by the light receiving unit 230 of the imaging processing device 200, and the light monitor value Qg, Qb detected by each color light monitor unit 150G, 150B.

For example, the red light source control unit 110R according to the present embodiment causes only the red light source 130R to turn on with a different light amount, and calculates the correlation between the luminance Lr detected by the light receiving unit 230 and the light monitor value Qr detected by the red light monitor unit 150R at that occasion. The green light source control unit 110G or the blue light source control unit 110B is configured to similarly calculate the correlation between the light monitor value Qg, Qb detected by the green light monitor unit 150G or blue light monitor unit 150B, and the luminance Lg, Lb detected by the light receiving unit 230. In other words, the green light source control unit 110G or the blue light source control unit 110B calculates the correlation between the light monitor value Qg (Qb) and the luminance Lg (Lb) with only the green light source 130G or the blue light source 130B being turned on with a different light amount.

For example, in the case of the endoscopic system according to the present embodiment, the correlations between the luminances Lr, Lg, and Lb, and the light monitor values Qr, Qg, and Qb are acquired for each of the RGB light sources at the time of white balance adjustment which is certainly performed after attachment of the endoscopic probe. Accordingly, it becomes possible to adjust the light amounts of respective emission light beams from the red light source 130R, the green light source 130G, and the blue light source 130B, in accordance with the luminances Lr_A, Lg_A, and Lb_A desired to be added to the radiated illumination light.

In the illuminating device 100 according to the present embodiment, the white light source 130W is turned on so that the luminance of light of each of the colors of R, G, and B in the radiated illumination light becomes equal to or lower than target values Lr_X, Lg_X, and Lb_X. The currents supplied to the red light source 130R, the green light source 130G, and the blue light source 130B are controlled, using, as target values, light monitor values Qr_A, Qg_A, and Qb_A equivalent to light amounts corresponding to the shortage luminances Lr_A, Lg_A, and Lb_A, respectively. In other words, once the luminances Lwr, Lwg, and Lwb of the light having respective colors of R, G, and B in the illumination light formed by white light emitted from the white light source 130W are grasped, the illuminating device 100 may adjust the luminances Lr, Lg, and Lb of respective colors of R, G, and B in the illumination light multiplexed with the white light to desired values.

### (1.1.2. Configuration example of imaging processing device)

The imaging processing device 200 includes an optical system 210, the light receiving unit 230, and an imaging processing unit 250. The optical system 210 takes in the illumination light radiated from the illuminating device 100. In the case of the endoscopic system according to the present embodiment, the optical system 210 may be capable of taking in the illumination light via an observation window attached to the tip of the endoscope probe.

The light receiving unit 230 includes a solid-state imaging element such as a charge coupled device (CCD) and a complementary metal oxide semiconductor (CMOS), for example. The light receiving unit 230 is disposed at an imaging position of the optical system 210, and a subject image of the target portion is captured by the illumination light radiated to and reflected by the target portion. The light receiving unit 230 generates an image signal by the photoelectric conversion of the captured subject image, and outputs the generated image signal to the imaging processing unit 250.

The imaging processing unit 250 includes a CPU and a storage element, generates an image on the basis of the image signal output from the light receiving unit 230, and displays the image on an un-illustrated monitor, or the like. At this time, the imaging processing unit 250 detects the luminance of the whole image or each pixel included in a preliminarily determined region. Furthermore, the imaging processing unit 250 calculates an average value of the luminance values detected in respective pixels, and outputs the calculated average value of the luminance values to the red light source control unit 110R, the green light source control unit 110G, and the blue light source control unit 110B in the illuminating device 100. At this time, in the endoscopic system according to the present embodiment, the luminance value detected by the light receiving unit 230 can be different depending on an individual difference of the endoscope probe.

### [1.2. Control processing example of illuminating device]

In the above, the overall configuration example of the image acquisition system 10 according to the present embodiment has been explained. Next, there will be explained control processing of the illuminating device 100 in the image acquisition system 10 according to the present embodiment.

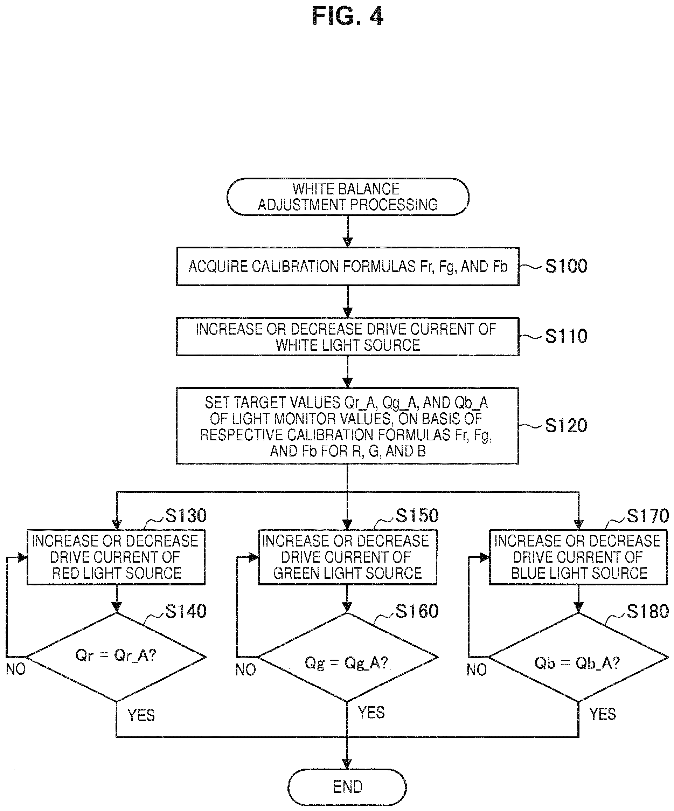

FIG. 4 illustrates a flowchart of a white balance adjustment processing example in the illuminating device 100 according to the present embodiment. This white balance adjustment processing is an example of adjusting the light amounts of the emission light beams from respective light sources so that the luminances of the light having respective colors of R, G, and B in the radiated illumination light turn out to be preliminarily set target values Lr_X, Lg_X, and Lb_X. This white balance adjustment processing flow may be started when an un-illustrated white balance adjustment processing start button is pressed down by a user, for example.

First, in step S100, the red light source control unit 110R, the green light source control unit 110G, and the blue light source control unit 110B calculate the correlations between the light monitor values Qr, Qg, and Qb of the emission light beams and the luminance values Lr, Lg, and Lb for the red light, the green light, and the blue light, respectively. In the present embodiment, calibration formulas Fr, Fg, and Fb are calculated expressing the correlations between the light monitor values Qr, Qg, and Qb of the emission light beams from the red light source 130R, the green light source 130G, and the blue light source 130B and the luminance values Lr, Lg, and Lb detected by the light receiving unit 230, respectively. The calculation of such calibration formulas Fr, Fg, and Fb may be performed in the stage of attaching the endoscope probe to the illuminating device 100 when the endoscopic system is started to be used, for example. By the calculation of such calibration formulas Fr, Fg, and Fb, the light monitor values Qr, Qg, and Qb of the emission light beams from the respective light sources and the luminances Lr, Lg, and Lb detected by the light receiving unit 230 are associated with each other respectively after the individual difference of the endoscope probe to be used is adjusted.

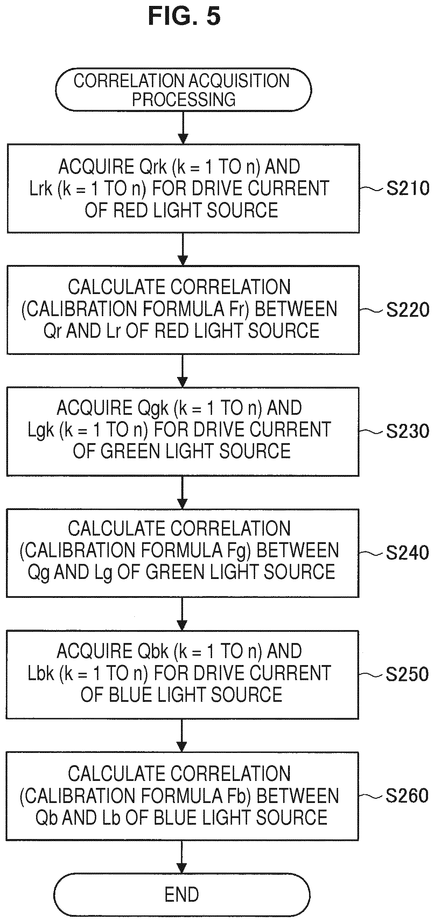

FIG. 5 illustrates a flowchart of correlation acquisition processing by the illuminating device 100. In the case of the endoscopic system according to the present embodiment, the following correlation acquisition processing is carried out under the condition that a cover is attached to the tip of the endoscope probe, for example, so as to image a white subject as a reference. Except for the case that the cover is attached to the tip of the endoscope probe, the correlation acquisition processing may be performed while imaging a preliminarily determined white subject.

First, in step S210, for the red light, the red light source control unit 110R measures the light monitor value (voltage value: V) Qr with respect to the drive current of the red light source 130R and the luminance Lr detected by the light receiving unit 230. Specifically, the red light source control unit 110R emits red light while changing the drive current supplied to the red light source 130R, in a state where the green light source 130G and the blue light source 130B are not turned on. During this state, the red light source control unit 110R acquires the light monitor value Qrₖ (k = 1 to n) detected by the red light monitor unit 150R and the luminance Lrₖ (k = 1 to n) detected by the light receiving unit 230 for a plurality of drive current values Aₖ (k = 1 to n).

Next, in step S220, the red light source control unit 110R calculates the calibration formula Fr expressing the correlation for the red light between the light monitor value Qr and the luminance Lr on the basis of the acquired light monitor value Qrₖ (k = 1 to n) and luminance Lrₖ (k = 1 to n). While a quadratic polynomial can be used for the calibration formula, for example, the calculation method of the calibration formula and the order of the calibration formula can be set arbitrarily.

After the calculation of the calibration formula Fr for the red light source 130R, subsequently in step S230, the green light source control unit 110G measures the light monitor value (V) with respect to the drive current of the green light source 130G and the luminance detected by the light receiving unit 230. Such measurement is performed while changing the drive current supplied to the green light source 130G in the state without causing the red light and the blue light to be emitted. Subsequently, in step S240, the green light source control unit 110G calculates the calibration formula Fg expressing the correlation for the green light on the basis of the acquired light monitor value Qgₖ (k = 1 to n) and luminance Lgₖ (k = 1 to n).

After the calculation of the calibration formula Fg for the green light source 130G, subsequently in step S250, the blue light source control unit 110B measures the light monitor value (V) with respect to the drive current of the blue light source 130B and the luminance detected by the light receiving unit 230. Such measurement is performed while changing the drive current supplied to the blue light source 130B in the state without causing the red light and the green light to be emitted. Next, in step S260, the blue light source control unit 110B calculates the calibration formula Fb expressing the correlation for the blue light on the basis of the acquired light monitor value Qbₖ (k = 1 to n) and luminance Lbₖ (k = 1 to n).

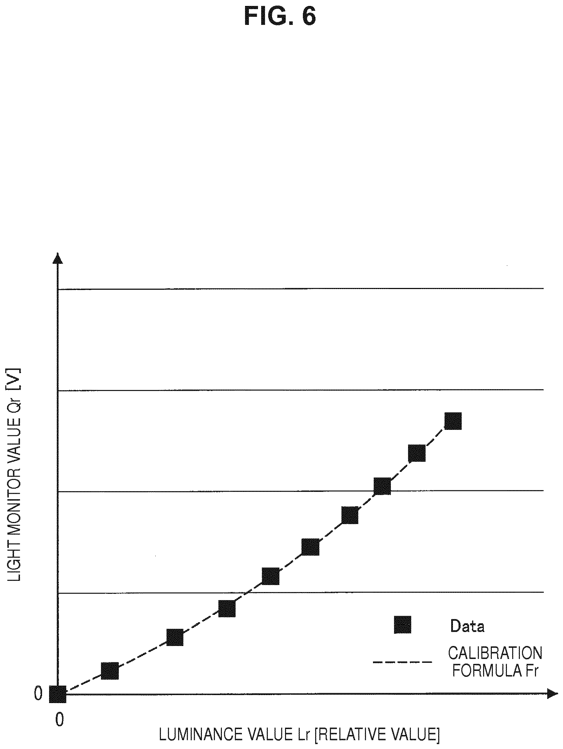

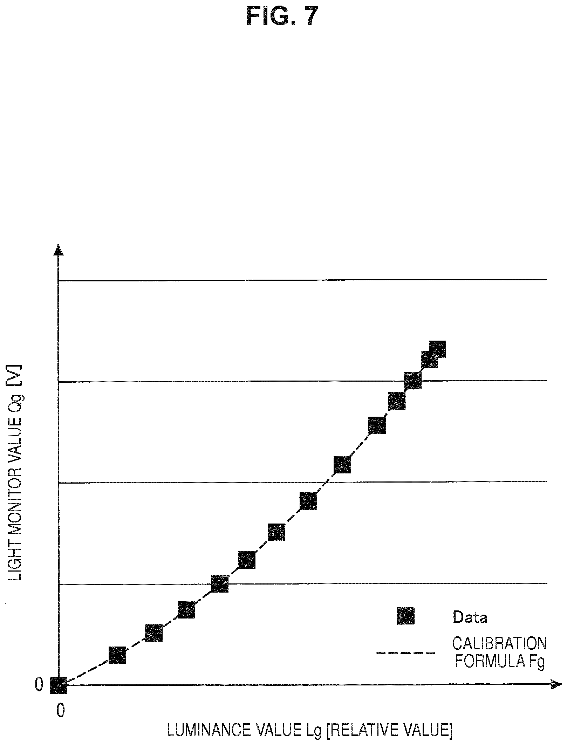

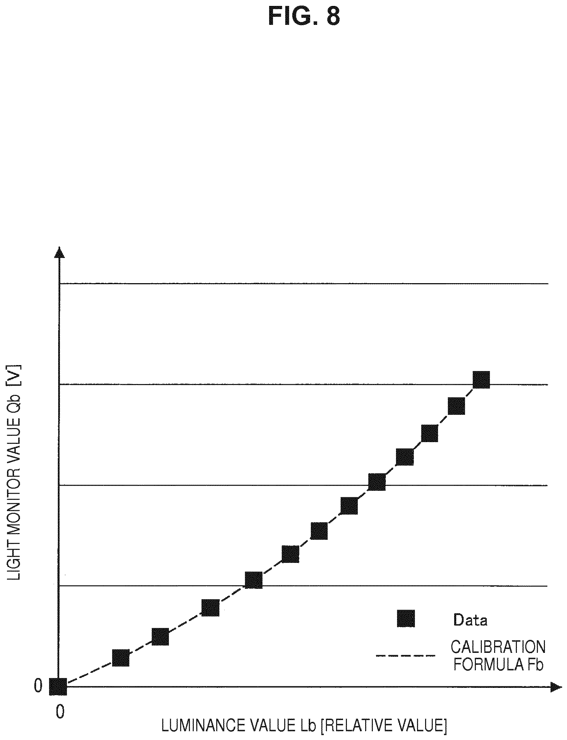

FIG. 6 to FIG. 8 illustrate the correlations between the light monitor values detected by the photodiodes and the luminances detected by the CCDs which are acquired in the above described correlation acquisition processing sequences for the red light, green light, and the blue light, respectively. Each of FIG. 6 to FIG. 8 has the same scale in the vertical axis and the horizontal axis. In the illustrated example, the gradients of the calibration formulas Fr, Fg, and Fb calculated from data acquired for the respective color light beams have the smallest value for the red light, the larger value for the blue light, and the largest value for the green light. That is, for the respective color light beams, the light monitor values Qr, Qg, and Qb to cause the luminances Lr, Lg, and Lb detected by the light receiving unit 230 to be the same have the smallest value for the red light, the larger value for the blue light, and the largest value for the green light.

Note that, while, in the correlation acquisition processing illustrated in FIG. 5, the calibration formulas Fr, Fg, and Fb are calculated in the order of the red light, the green light, and the blue light, the order may be changed arbitrarily. Further, the calibration formulas Fr, Fg, and Fb may be calculated for the respective color light beams after the acquisition of the light monitor values and the luminances for all of the light sources of the three colors.

Returning to FIG. 4, after each control unit has acquired the calibration formulas Fr, Fg, and Fb relating to the light emitted from respective light sources in step S100, the process flow proceeds to step S110. In step S110, the white light source control unit 110W increases or decreases the drive current of the white light source 130W, in a state where the red light source 130R, the green light source 130G, and the blue light source 130B are not turned on. On this occasion, the drive current is set so that the luminances of the light having respective colors of R, G, and B detected by the light receiving unit 230 do not exceed the target values Lr_X, Lg_X, and Lb_X, respectively. The target values Lr_X, Lg_X, and Lb_X of the luminances of respective colors of R, G, and B at the time of white balance adjustment may be set to 200 (cd/m²), for example.

Next, in step S120, the red light source control unit 110R calculates the luminance Lr_A of the red light falling short with respect to the target value Lr_X of the luminance of the red light in the illumination light. In addition, the red light source control unit 110R calculates the light monitor value Qr_A corresponding to the luminance Lr_A, on the basis of the calibration formula Fr. The red light source control unit 110R then sets the calculated value Qr_A as the target value of drive control of the red light source 130R.

In step S120, the green light source control unit 110G or the blue light source control unit 110B similarly calculates the luminances Lg_A or Lb_A falling short with respect to the target value Lg_X, Lb_X of the luminance. In addition, the green light source control unit 110G or the blue light source control unit 110B calculates the light monitor value Qg_A, Qb_A corresponding to the luminance Lg_A, Lb_A, on the basis of the calibration formula Fg, Fb, and sets the calculated value as the target value of drive control of the green light source 130G or the blue light source 130B.

Next, the red light source control unit 110R increases or decreases the drive current of the red light source 130R in step S130. Subsequently, the red light source control unit 110R determines whether or not the light monitor value Qr detected by the red light monitor unit 150R has become the target value Qr_A set in step S120, in step S140. The red light source control unit 110R repeats the processing of step S130 to step S140 until the detected light monitor value Qr becomes the target value Qr_A.

Similarly, also the green light source control unit 110G and the blue light source control unit 110B repeat the increase or decrease of the drive currents and the determinations until the light monitor values Qg and Qb detected by the green light monitor unit 150G and the blue light monitor unit 150B become the target values Qg_A and Qb_A set in step S120 (step S150 to step S160 and step S170 to step S180), respectively.

When the light monitor values Qr, Qg, and Qb coincide with the target values Qr_A, Qg_A, and Qb_A for all of the red light, the green light, and the blue light, the white balance adjustment processing is finished. As thus described, the endoscopic system according to the present embodiment can easily perform white balance adjustment without referring to images acquired by the imaging processing device 200 after the calibration formulas Fr, Fg, and Fb for the light monitor values and luminances of respective emission light beams from the RGB light sources have once been acquired.

As explained above, in the illuminating device 100 and the image acquisition system 10 according to the first embodiment of the present disclosure, the light amount of the red light emitted from the red light source 130R is detected by the red light monitor unit 150R as the light monitor value Qr. Similarly, in the illuminating device 100 and the image acquisition system 10, the light amounts of the green light and the blue light emitted from the green light source 130G and the blue light source 130B are detected by the green light monitor unit 150G and the blue light monitor unit 150B as the light monitor values Qg and Qb, respectively.

In addition, the red light source control unit 110R, the green light source control unit 110G and the blue light source control unit 110B in the illuminating device 100 and the image acquisition system 10 described above receive, together with the respective light monitor values Qr, Qg, and Qb, the luminances Lr, Lg, and Lb detected by the light receiving unit 230 of the imaging processing device 200. The red light source control unit 110R, the green light source control unit 110G, and the blue light source control unit 110B then acquire calibration formulas Fr, Fg, and Fb indicating the correlations between the luminances Lr, Lg, and Lb, and the light monitor values Qr, Qg, and Qb.

Therefore, the illuminating device 100 and the image acquisition system 10 described above can multiplex respective emission light beams with white light and adjust white balance of the illumination light while adjusting the light amounts of the emission light beams from the red light source 130R, the green light source 130G, and the blue light source 130B, on the basis of the calibration formulas Fr, Fg, and Fb. Accordingly, it becomes unnecessary to adjust the gain of the light receiving unit 230 of the imaging processing device 200, whereby high-quality images can be acquired with reduced electronic noise. In addition, adjusting the white balance of the illumination light by adjusting the light amounts of RGB light sources instead of adjusting the gain of the light receiving unit 230 allows for reducing power consumption of respective light sources. In addition, adjusting the white balance of the illumination light by adjusting the light amounts of the RGB light sources instead of adjusting the gain of the light receiving unit 230 allows for accurately adjusting the white balance of the illumination light without referring to images acquired by the imaging processing device 200 each time.

In addition, the illuminating device 100 and the image acquisition system 10 described above calculate the calibration formulas Fr, Fg, and Fb expressing the correlations between the respective light monitor values Qr, Qg, and Qb, and the luminances Lr, Lg, and Lb detected by the light receiving unit 230, when for example attaching the endoscopic probe. Accordingly, thereafter, the white balance of the illumination light can be easily adjusted without taking into account the influence of individual differences among the endoscopic probes, as long as the same endoscopic probe is kept using. Furthermore, it is possible, even when different endoscopic probes are used, to accurately adjust the white balance of the illumination light by calculating the calibration formulas Fr, Fg, and Fb when attaching respective endoscopic probes. Therefore, a desired captured image suitable for the imaging object can be acquired regardless of individual differences among endoscopic probes in an endoscopic system used in the medical front, for example.

In addition, the illuminating device 100 and the image acquisition system 10 described above have two white light sources 130W respectively capable of radiating white light, and RGB light sources. Therefore, even in a case where one of the light sources fails, it is possible to safely take out the endoscope from the body while radiating white light of a desired luminance from the other light source, and either of the light sources can function as an emergency light.

Note that, usually, although the target values Lr_X, Lg_X, and Lb_X of the luminances of the light having respective colors of R, G, and B in the illumination light are assumed to be the same value in white balance adjustment, the aforementioned control processing may be performed for different target values of the luminances of the light having respective colors of R, G, and B in the illumination light. In addition, in the illuminating device 100 and the image acquisition system 10 according to the present embodiment, the white light source 130W, as long as it emits white light, the luminance of which does not exceed the target values of the luminances of the light having respective colors of R, G, and B in the radiated illumination light, may be a lamp light source such as a xenon lamp or a Halogen lamp. In other words, the light amount of the white light source 130W need not be controllable when the light amount of the white light emitted from the white light source 130W is slightly small. In such a case, too, the shortages of the luminances of the light having respective colors of R, G, and B in the illumination light are supplemented by the red light source 130R, the green light source 130G, and the blue light source 130B, respectively.

### <2. Second embodiment>

A second embodiment of the present disclosure is different from the first embodiment in the point that a white light source, a red light source, a green light source, and a blue light source are driven and controlled by a common control unit. In the following, the point different from the illuminating device of the first embodiment will be explained.

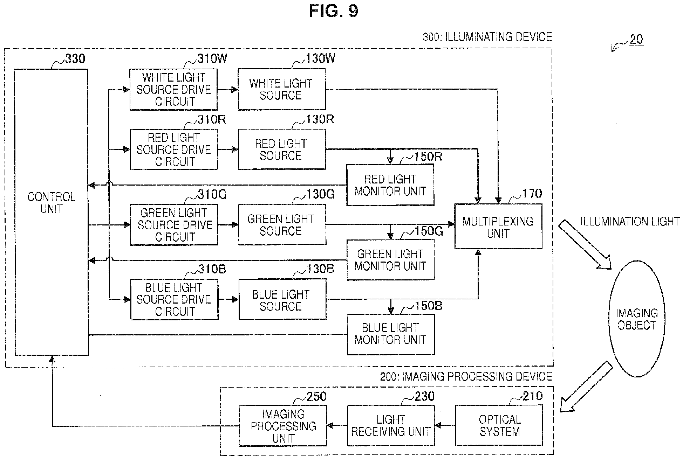

FIG. 9 is a block diagram illustrating an overall configuration of an image acquisition system 20 according to the second embodiment of the present disclosure. Similarly to the image acquisition system 10 of the first embodiment, this image acquisition system 20 includes an illuminating device 300 and an imaging processing device 200 and is configured as an endoscopic system, for example. In this configuration, the imaging processing device 200 can be configured similarly to the imaging processing device 200 of the image acquisition system 10 according to the first embodiment except the point that a signal is output from an imaging processing unit 250 to a control unit 330 of the illuminating device 300.

The illuminating device 300 includes a white light source 130W, a red light source 130R, a green light source 130G, a blue light source 130B, a red light source drive circuit 310R, a green light source drive circuit 310G, a blue light source drive circuit 310B, the control unit 330, and a multiplexing unit 170. Further, the illuminating device 300 includes a red light monitor unit 150R, a green light monitor unit 150G, and a blue light monitor unit 150B.

The white light source 130W, the red light source 130R, the green light source 130G, and the blue light source 130B can be configured similarly to the light source in the illuminating device 100 according to the first embodiment. Further, the light source is not limited to the light sources for three colors R, G, and B and the number of light sources is not limited as in light sources for four colors or the like. The red light monitor unit 150R, the green light monitor unit 150G, and the blue light monitor unit 150B can be configured similarly to the respective light monitor units of the illuminating device 100 according to the first embodiment except that detected light monitor values Qr, Qg, and Qb are transmitted to the control unit 330. Also the multiplexing unit 170 can be configured similarly to the multiplexing unit 170 of the illuminating device 100 exemplified in FIG. 3 according to the first embodiment.

The control unit 330 controls the drive currents of the white light source 130W, the red light source 130R, the green light source 130G, and the blue light source 130B. For the red light source 130R, the green light source 130G, and the blue light source 130B, the drive currents are set for respective light sources on the basis of the correlations between the respective luminances Lr, Lg, and Lb of the red light, green light, and the blue light detected by the light receiving unit 230 of the imaging processing device 200, and the light monitor values Qr, Qg, and Qb detected by the respective light monitor units. The control unit 330 transmits drive commands for the respective light sources to a white light source drive circuit 310W, the red light source drive circuit 310R, the green light source drive circuit 310G, and the blue light source drive circuit 310B, on the basis of the drive currents set for respective light sources. The drive circuits of respective light sources drive the white light source 130W, the red light source 130R, the green light source 130G, and the blue light source 130B, respectively, on the basis of the drive commands.

In the illuminating device 100 according to the first embodiment, the red light source control unit 110R, the green light source control unit 110G, and the blue light source control unit 110B receive the light monitor values Qr, Qg, and Qb and the luminances Lr, Lg, and Lb and calculate the calibration formulas Fr, Fg, and Fb expressing the correlations, respectively. On the other side, in the illuminating device 300 according to the present embodiment, the common control unit 330 is configured to receive the light monitor values Qr, Qg, and Qb and the luminances Lr, Lg, and Lb and to calculate the calibration formulas Fr, Fg, and Fb expressing the correlations between the light monitor values and the luminances, respectively. The contents of a calculation method of such calibration formulas Fr, Fg, and Fb, and the contents of the white balance adjustment processing to be performed by the control unit 330 can be carried out similarly to the illuminating device 100 according to the first embodiment.

The illuminating device 300 and the image acquisition system 20 according to the present embodiment can provide the same effect as the illuminating device 100 and the image acquisition system 10 according to the first embodiment. In addition, in the illuminating device 300 and the image acquisition system 20 according to the present embodiment, the target value calculation of the light monitor values in driving and controlling the respective light sources is performed by one control unit, and it is possible to reduce the load in the transmission and reception of the calculation results and the like between the control units. Note that, while the control unit 330 is provided in the illuminating device 300 in the example illustrated in FIG. 9, the control unit 330 may be provided as a control device separated from the illuminating device 300, and the imaging processing device 200 may include a control unit.

### <3. Third embodiment>

A third embodiment of the present disclosure differs from the first embodiment in that not only the red light source, the green light source, and the blue light source, but also the white light source is controlled, on the basis of the correlation between the light amount detected by the light monitor unit and the luminance detected by the light receiving unit. In the following, description will be provided mainly on different points from the illuminating device according to the first embodiment.

### [3.1. Overall configuration example of image acquisition system]

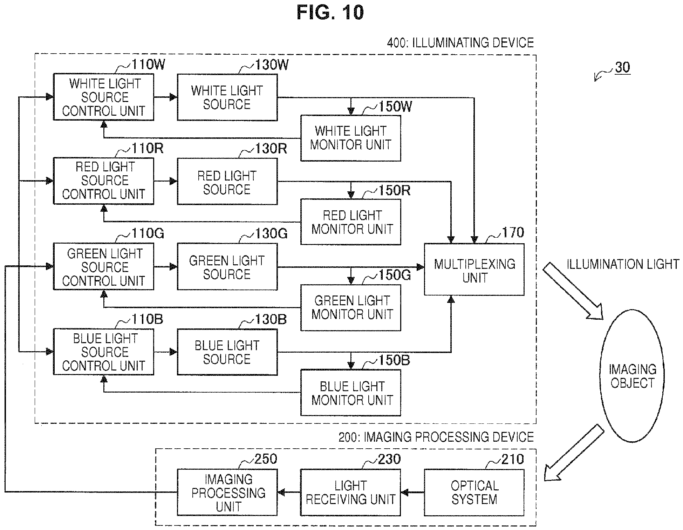

FIG. 10 is a block diagram illustrating the overall configuration of an image acquisition system 30 according to the present embodiment. The image acquisition system 10 includes an illuminating device 400 and an imaging processing device 200 similarly to the image acquisition system 10 of the first embodiment, and is configured for example as an endoscopic system. Among these, the imaging processing device 200 may be configured similarly to the imaging processing device 200 of the image acquisition system 10 according to the first embodiment except for outputting signals also to the white light source control unit 110W.

The illuminating device 400 includes a white light source 130W, a red light source 130R, a green light source 130G, a blue light source 130B, a red light source control unit 110R, a green light source control unit 110G, a blue light source control unit 110B, and a multiplexing unit 170. The illuminating device 400 further includes a white light monitor unit 150W, a red light monitor unit 150R, a green light monitor unit 150G, and a blue light monitor unit 150B.

The white light source 130W, the red light source 130R, the green light source 130G, and the blue light source 130B can be configured similarly to the light sources of the illuminating device 100 according to the first embodiment. In the illuminating device 400 according to the present embodiment, however, also for the white light source 130W, it is possible to control the light amount of the white light by increasing or decreasing the supply current. In addition, the light source is not limited to the light sources for three colors R, G, and B but may be light sources for four colors or the like, and the number of light sources is not limited. The red light monitor unit 150R, the green the light monitor unit 150G, and the blue light monitor unit 150B can be configured similarly to the light monitor unit of the illuminating device 100 according to the first embodiment. In addition, the white light monitor unit 150W, which is also formed using photodiodes or the like similarly to other light monitor units, receives a part of the white light emitted from the white light source 130W, converts the light amount of the received light into a voltage signal, and transmits the signal to the white light source control unit 110W.

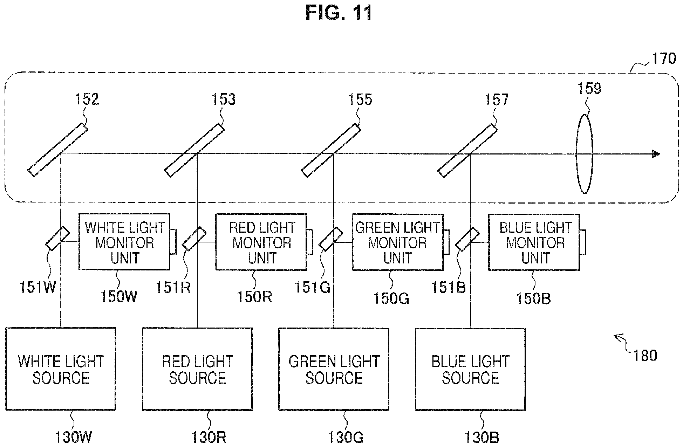

FIG. 11 illustrates a configuration example of the multiplexing module 180 with a light monitor, including the multiplexing unit 170 of the illuminating device 400 according to the present embodiment. The multiplexing module 180 described above can be configured similarly to the multiplexing module 180 of the illuminating device 100 according to the first embodiment illustrated in FIG. 3 except the point that the white light monitor unit 150W and the light sampler 151W are added thereto.

The red light source control unit 110R, the green light source control unit 110G, and the blue light source control unit 110B can be basically configured similarly to the control units of the respective light sources of the illuminating device 100 according to the first embodiment. In other words, the control unit of each of the RGB light sources calculates the correlation (calibration formula) between the luminance Lr (Lg, Lb) detected by the light receiving unit 230 of the imaging processing device 200 and the light monitor value Qr (Qg, Qb) detected by each light monitor unit. In addition, the control unit of each of the RGB light sources controls the drive current of each light source on the basis of the calculated correlation.

In addition, the white light source control unit 110W in the present embodiment also controls the drive current supplied to the white light source 130W, on the basis of the correlations between the luminances Lwr, Lwg, and Lwb detected by the light receiving unit 230 of the imaging processing device 200, and the light monitor value Qw detected by the white light monitor unit 150W. The white light source control unit 110W turns on only the white light source 130W with a different light amount, and calculates the correlations between the luminances Lwr, Lwg, and Lwb of the light having respective colors of R, G, and B detected by the light receiving unit 230 on that occasion, and the light monitor value Qw of the white light detected by the white light monitor unit 150W. In other words, the white light source control unit 110W calculates three correlations corresponding to respective colors of R, G, and B. Note that the light receiving unit 230 is capable of dispersing the received white light into light having respective colors of R, G, and B, and detecting the luminances Lwr, Lwg, and Lwb of the light having respective colors.

For example, in the case of the endoscopic system according to the present embodiment, the correlations between the luminances Lr, Lg, and Lb, and the light monitor values Qr, Qg, and Qb are acquired for each of the RGB light sources at the time of white balance adjustment which is certainly performed after attachment of the endoscopic probe. On this occasion, the correlations between the light monitor value Qw and the luminances Lwr, Lwg, and Lwb of the light having respective colors of R, G, and B are also acquired respectively for the white light source 130W. Accordingly, thereafter, adjustment of the color temperature and the light amount of the illumination light can be accurately performed without referring to the luminance or color temperature detected by the imaging processing device 200.

In the illuminating device 400 according to the present embodiment, the white light source 130W is turned on with a light monitor value Qw at which the luminances of the light having respective colors of R, G, and B in the radiated illumination light all become equal to or lower than the target values, on the basis of the correlations between the light monitor value Qw of the white light and the luminances Lwr, Lwg, and Lwb. Subsequently, the light monitor values Qr, Qg, and Qb equivalent to the light amounts corresponding to the shortage luminances Lr, Lg, and Lb are calculated on the basis of the correlations, thereby controlling the current supplied to the red light source 130R, the green light source 130G, and the blue light source 130B. Accordingly, it becomes possible to precisely adjust the luminances Lr, Lg, and Lb of the illumination light, without referring each time to the luminances Lr, Lg, and Lb of the radiated illumination light.

### [3.2. Control processing example of illuminating device]

In the above, the overall configuration example of the image acquisition system 30 according to the present embodiment has been explained. Next, there will be explained control processing of the illuminating device 100 in the image acquisition system 30 according to the present embodiment.

### (3.2.1. White balance adjustment processing example)

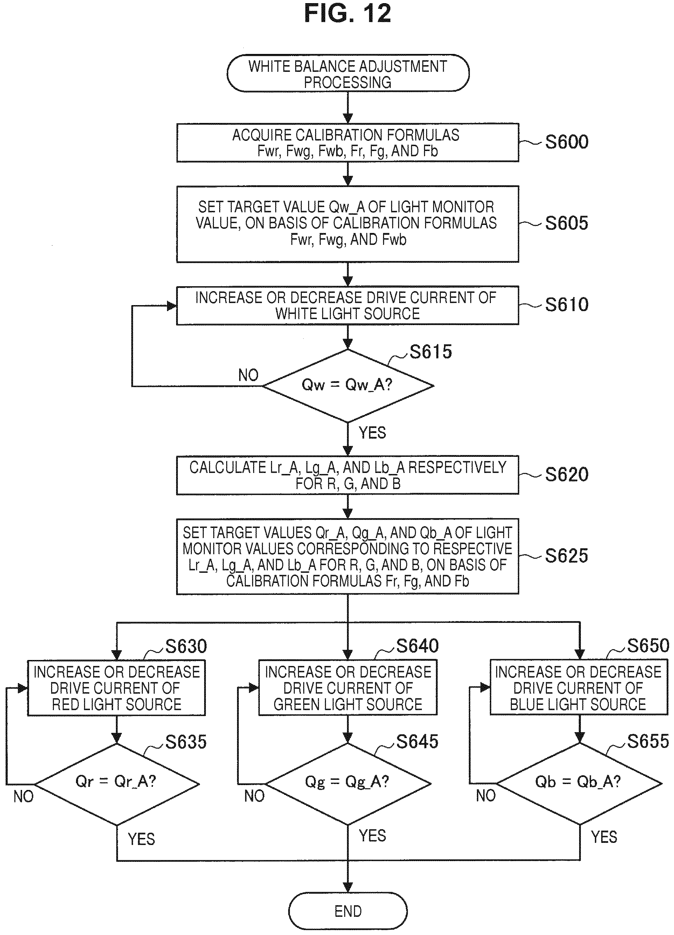

FIG. 12 illustrates a flowchart of a white balance adjustment processing example in the illuminating device 400 according to the present embodiment. This white balance adjustment processing is an example of adjusting the light amounts of the emission light beams from respective light sources so that the luminances of the light having respective colors of R, G, and B in the radiated illumination light turn out to be preliminarily set target values Lr_X, Lg_X, and Lb_X. This white balance adjustment processing flow may be started when an un-illustrated white balance adjustment processing start button is pressed down by a user, for example.

First, in step S600, the white light source control unit 110W calculates, respectively, the correlations between the light monitor value Qw of the white light and the luminances Lwr, Lwg, and Lwb of the light having respective colors of R, G, and B. Here, the calibration formulas Fwr, Fwg, and Fwb expressing the correlations between the light monitor value Qw of the white light emitted from the white light source 130W, and the luminances Lwr, Lwg, and Lwb detected by the light receiving unit 230 are calculated, respectively. Additionally, in step S600, the red light source control unit 110R, the green light source control unit 110G, and the blue light source control unit 110B calculate the correlations between the light monitor values Qr, Qg, and Qb of the emission light beams, and the luminances Lr, Lg, and Lb for the red light, the green light, and the blue light, respectively. Similarly to the first embodiment, the calibration formulas Fr, Fg, and Fb expressing the correlations between the light monitor values Qr, Qg, and Qb of the emission light beams from the red light source 130R, the green light source 130G, and the blue light source 130B, and the luminances Lr, Lg, and Lb detected by the light receiving unit 230 are calculated, respectively.

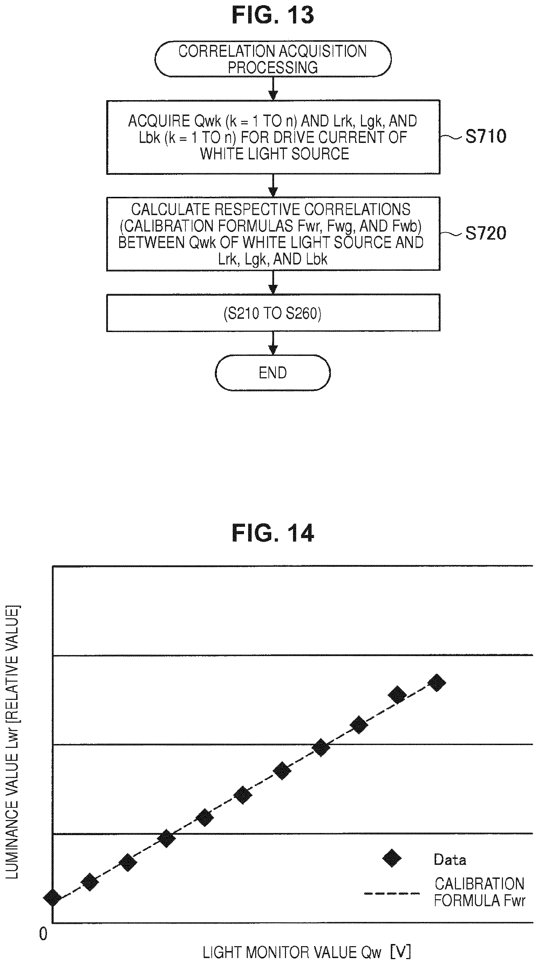

FIG. 13 illustrates a flowchart of correlation acquisition processing by the illuminating device 400. Similar to the present embodiment, the following correlation acquisition processing is carried out under the condition that a cover is attached to the tip of the endoscope probe, for example, so as to image a white subject as a reference. Except for the case that the cover is attached to the tip of the endoscope probe, the correlation acquisition processing may be performed while imaging a preliminarily determined white subject.

First, in step S710, for the white light, the white light source control unit 110W measures the light monitor value (voltage value: V) Qw with respect to the drive current of the white light source 130W and the luminances Lwr, Lwg, and Lwb of the light having respective colors of R, G, and B detected by the light receiving unit 230. Specifically, the white light source control unit 110W emits white light while changing the drive current supplied to the white light source 130W in the state where the red light source 130R, the green light source 130G, and the blue light source 130B are not turned on. During this state, the white light source control unit 110W acquires the light monitor value Qwₖ (k = 1 to n) detected by the white light monitor unit 150W and the luminances Lwrₖ, Lwgk, and Lwbk (k = 1 to n) of the light having respective colors of R, G, and B detected by the light receiving unit 230 for a plurality of drive current values Aₖ (k = 1 to n).

Next, in step S720, the white light source control unit 110W calculates the calibration formulas Fwr, Fwg, and Fwb expressing the correlations between the light monitor value Qw and the luminances Lwr, Lwg, and Lwb, on the basis of the acquired light monitor value Qwₖ (k = 1 to n) and the luminances Lwrₖ, Lwgₖ, and Lwbₖ (k = 1 to n). While a quadratic polynomial can be used for the calibration formula, for example, the calculation method of the calibration formula and the order of the calibration formula can be set arbitrarily.

After the calculation of the calibration formulas Fwr, Fwg, and Fwb for the white light source 130W, the calibration formulas Fwr, Fwg, and Fwb are calculated also for the red light source 130R, the green light source 130G, and the blue light source 130B in accordance with the procedure described in the first embodiment, (steps S210 to S260).

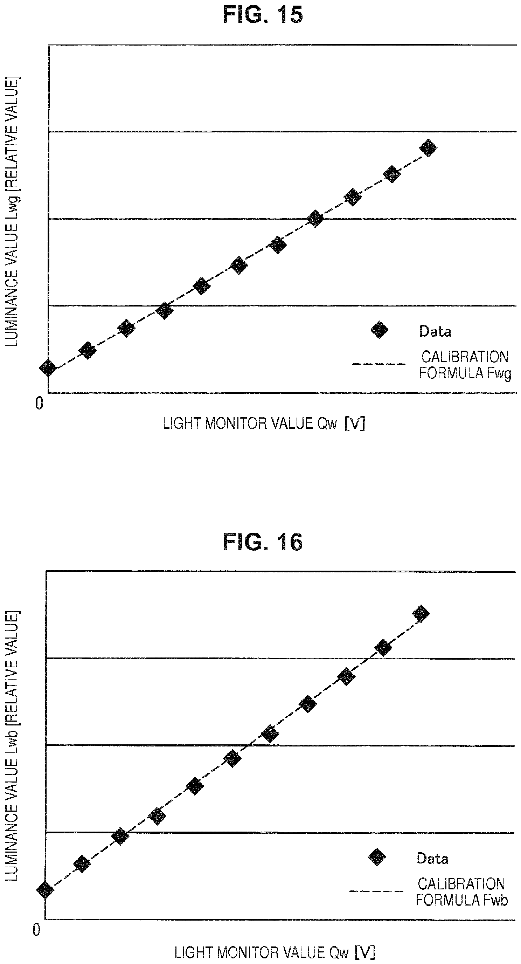

FIG. 14 to FIG. 16 illustrate, for the white light, the correlations between the light monitor value Qw detected by a photodiode, and the luminances Lwr, Lwg, and Lwb detected by a CCD in the aforementioned procedure of correlation acquisition processing. In FIG. 14 to FIG. 16, the horizontal axis indicates the light monitor value and the vertical axis indicates the luminance. In addition, the vertical axes in FIG. 14 to FIG. 16 are drawn in the same scale each other, and the horizontal axes in FIG. 14 to FIG. 16 are drawn in the same scale each other.

In the example illustrated in FIG. 14 to FIG. 16, the luminance Lwr of the red light has the smallest gradient, followed by the luminance Lwg of the green light with a larger gradient, and the luminance Lwb of the blue light with the largest gradient, according to the calibration formulas Fwr, Fwg, and Fwb calculated from the acquired data. In other words, when the white light source 130W is turned on, the luminance Lwr of the red light in the illumination light is the smallest, followed by the larger luminance Lwg of the green light, and the largest luminance Lwb of the blue light.

Note that, although the calibration formulas Fwr, Fwg, Fwb, Fr, Fg, and Fb are calculated in the order of the white light, the red light, the green light, and the blue light in the correlation acquisition processing illustrated in FIG. 13, the order may be changed arbitrarily. In addition, the calibration formulas Fwr, Fwg, Fwb, Fr, Fg, and Fb for respective light sources may be calculated after acquisition of the light monitor values and the luminance values for all of the light sources.

Returning to FIG. 12, after acquisition, by each control unit, of the calibration formulas Fwr, Fwg, Fwb, Fr, Fg, and Fb relating to the light emitted from respective light sources in step S600, the process flow proceeds to step S605. In step S605, the white light source control unit 110W calculates a light monitor value Qw_A at which the luminances of the light having respective colors of R, G, and B all become equal to or lower than the preliminarily set target luminance values Lr_X, Lg_X, and Lb_X, on the basis of the calibration formulas Fwr, Fwg, and Fwb. The white light source control unit 110W then sets the calculated light monitor value Qw_A as the target value of drive control of the white light source 130W. The target values Lr_X, Lg_X, and Lb_X of the luminances of respective colors of R, G, and B at the time of white balance adjustment may be 200 (cd/m²), for example.

Next, the white light source control unit 110W increases or decreases the drive current of the white light source 130W in step S610. Subsequently, in step S615, the white light source control unit 110W determines whether or not the light monitor value Qw detected by the white light monitor unit 150W has become the target value Qw_A set in step S605. The white light source control unit 110W repeats the processing of step S610 to step S615 until the detected light monitor value Qw becomes the target value Qw_A.

Next, in step S620, the red light source control unit 110R calculates a difference Lr_A between the target luminance Lr_X of the red light in the illumination light and the luminance Lr of the red light corresponding to the light monitor value Qw_A of the current white light. In step S620, also for the green light source control unit 110G or the blue light source control unit 110B, difference Lg_A, Lb_A is similarly calculated between the target luminance Lg_X, Lb_X of the light having respective colors in the illumination light, and the luminance Lg, Lb of the light having respective colors corresponding to the light monitor value Qw_A of the current white light.

Next, in step S625, the red light source control unit 110R calculates the light monitor value Qr_A corresponding to the aforementioned difference Lr_A of the luminance, on the basis of the calibration formula Fr. The red light source control unit 110R then sets the calculated value Qr_A as the target value of drive control of the red light source 130R. In step S625, also the green light source control unit 110G or the blue light source control unit 110B similarly sets the light monitor value Qg_A, Qb_A to be the target value of drive control of the green light source 130G or the blue light source 130B, on the basis of the calibration formula Fg, Fb.

Next, the red light source control unit 110R increases or decreases the drive current of the red light source 130R in step S630. Subsequently, the red light source control unit 110R determines whether or not the light monitor value Qr detected by the red light monitor unit 150R has become the target value Qr_A set in step S625, in step S635. The red light source control unit 110R repeats the processing of step S630 to step S635 until the detected light monitor value Qr becomes the target value Qr_A.

Similarly, also the green light source control unit 110G and the blue light source control unit 110B repeat the increase or decrease of the drive currents and the determinations until the light monitor values Qg and Qb detected by the green light monitor unit 150G and the blue light monitor unit 150B become the target values Qg_A and Qb_A set in step S120 (step S640 to step S645 and step S650 to step S655), respectively.

The white balance adjustment processing terminates when the light monitor values Qr, Qg, and Qb coincide with the target values Qr_A, Qg_A, and Qb_A for all of the red light source 130R, the green light source 130G and the blue light source 130B. As thus described, the endoscopic system according to the present embodiment acquires the calibration formulas Fwr, Fwg, Fwb, Fr, Fg, and Fb expressing the correlations between the light monitor values and the luminances of the emission light beams of the white light source and the RGB light sources after attachment of the endoscopic probe, for example. Accordingly, thereafter, white balance adjustment can be easily performed without referring to images acquired by the imaging processing device 200.

Note that, in the flow of the white balance adjustment processing illustrated in FIG. 12, although the luminance of the illumination light is corrected in accordance with the emission light beams emitted from the RGB light sources, mainly based on the white light emitted from the white light source 130W, the white balance adjustment processing is not limited to such an example. The luminance of the illumination light may be corrected by the white light emitted from the RGB light sources mainly based on the emission light beams emitted from the white light source 130W.

### (3.2.2. Color temperature adjustment processing example)

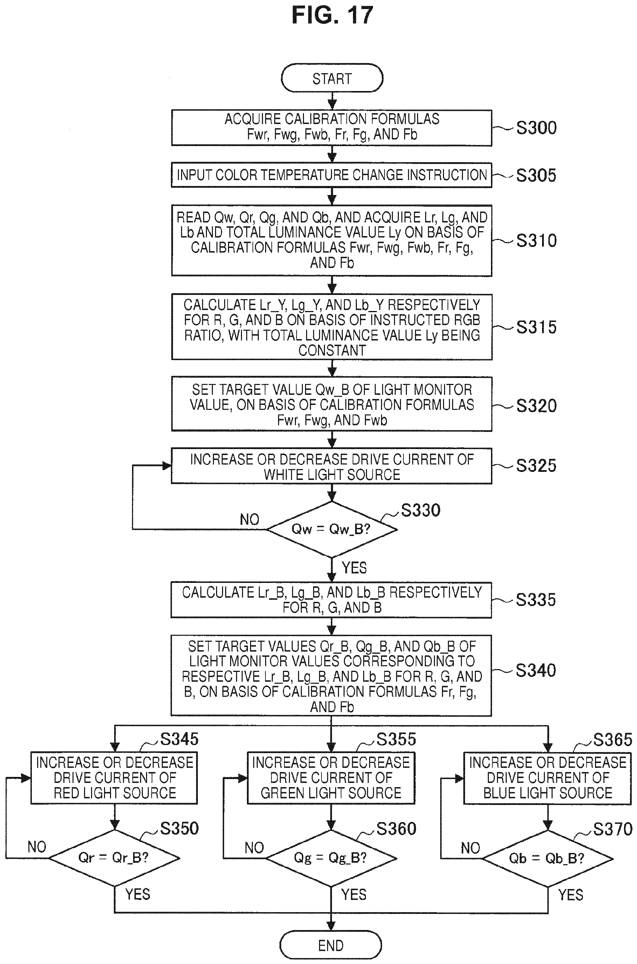

FIG. 17 illustrates a flowchart of a color temperature adjustment processing example in the illuminating device 400 according to the present embodiment. This color temperature adjustment processing flow is a processing flow to adjust the color temperature of the illumination light by changing the luminance ratio of the red light, the green light, and the blue light (RGB ratio) while maintaining the light amount (Qx) of the currently radiated illumination light.

First, in step S300, for the white light, the white light source control unit 110W calculates the correlations between the light monitor value Qw of the emission light beams and the luminances Lwr, Lwg, and Lwb of the light having respective colors of R, G, and B. In addition, the red light source control unit 110R, the green light source control unit 110G, and the blue light source control unit 110B calculate the correlations between the light monitor values Qr, Qg, and Qb of the emission light beams, and the luminances Lr, Lg, and Lb, for the red light, the green light, and the blue light, respectively. This step S300, which is performed in a procedure illustrated in FIG. 13 similarly to step S600 of the white balance adjustment processing illustrated in FIG. 12, may be performed in the stage of attaching the endoscopic probe to the illuminating device 400.

Next, in step S305, the white light source control unit 110W, the red light source control unit 110R, the green light source control unit 110G, and the blue light source control unit 110B receive an input of an instruction to change the color temperature of the illumination light. The instruction to change the color temperature in step S305 is input by a user, for example, when he/she sets an RGB ratio or presses an un-illustrated input switch having an RGB ratio preliminarily set thereto.

Next, in step S310, the control units of respective light sources read the light monitor values Qw, Qr, Qg, and Qb detected by the white light monitor unit 150W, the red light monitor unit 150R, the green light monitor unit 150G, and the blue light monitor unit 150B, respectively. The control units of respective light sources may exchange information of the light monitor values Qw, Qr, Qg, and Qb, or the control units of respective light sources may read all of the light monitor values Qw, Qr, Qg, and Qb, respectively. The respective control units then calculate the respective luminances Lr, Lg, and Lb of the light having respective colors of R, G, and B corresponding to the light monitor values Qw, Qr, Qg, and Qb, together with their total luminance value Ly, on the basis of the previously calculated calibration formulas Fwr, Fwg, Fwb, Fr, Fg, and Fb.

For example, the luminance Lr of the red light in the illumination light is calculated as the sum of the luminance value acquired from the light monitor value Qr on the basis of the calibration formulas Fr, and the luminance value acquired from the light monitor value Qw on the basis of the calibration formula Fwr. The luminance Lg of the green light in the illumination light is also calculated on the basis of the calibration formulas Fg and Fwg. Similarly, the luminance Lb of the blue light in the illumination light is calculated on the basis of the calibration formulas Fb and Fwb.

In addition, the total luminance value Ly is calculated by addition of the respectively acquired luminances Lr, Lg, and Lb. Calculation of the total luminance value Ly may be performed by any of the white light source control unit 110W, the red light source control unit 110R, the green light source control unit 110G, and the blue light source control unit 110B, and the total luminance value Ly may be output to the other control units.

Next, in step S315, the white light source control unit 110W calculates the target luminances Lr_Y, Lg_Y, and Lb_Y of respective colors of R, G, and B in the illumination light, on the basis of the instructed RGB ratio, while maintaining the total luminance value Ly. Calculation of such target luminances Lr_Y, Lg_Y, and Lb_Y may be performed by the control unit of another light source. Next, in step S320, the white light source control unit 110W calculates the light monitor value Qw_B at which the luminances of the light having respective colors of R, G, and B all become equal to or lower than their respective target luminances Lr_Y, Lg_Y, and Lb_Y, on the basis of the calibration formulas Fwr, Fwg, and Fwb. The white light source control unit 110W then sets the calculated light monitor value Qw_B as the target value of drive control of the white light source 130W.

Next, in step S325, the white light source control unit 110W increases or decreases the drive current of the white light source 130W. Subsequently, in step S330, the white light source control unit 110W determines whether or not the light monitor value Qw detected by the white light monitor unit 150W has become the target value Qw_B set in step S320. The white light source control unit 110W repeats the processing of step S325 to step S330 until the detected light monitor value Qw becomes the target value Qw_B.

Next, in step S335, the red light source control unit 110R calculates a difference Lr_B between the target luminance Lr_Y of the red light in the illumination light and the luminance Lr of the red light corresponding to the light monitor value Qw_B of the current white light. In step S335, also for the green light source control unit 110G or the blue light source control unit 110B, difference Lg_B, Lb_B is similarly calculated between the target luminance Lg_Y, Lb_Y of the light having respective colors in the illumination light, and the luminance Lg, Lb of the light having respective colors corresponding to the light monitor value Qw_B of the current white light.

Next, in step S340, the red light source control unit 110R calculates the light monitor value Qr_B corresponding to the aforementioned difference Lr_B of the luminance, on the basis of the calibration formula Fr. The red light source control unit 110R then sets the calculated value Qr_B as the target value of drive control of the red light source 130R. In step S340, also the green light source control unit 110G or the blue light source control unit 110B similarly sets the light monitor value Qg_B, Qb_B to be the target value of drive control of the green light source 130G or the blue light source 130B, on the basis of the calibration formula Fg, Fb.

Next, the red light source control unit 110R increases or decreases the drive current of the red light source 130R in step S345. Subsequently, the red light source control unit 110R determines whether or not the light monitor value Qr detected by the red light monitor unit 150R has become the target value Qr_B set in step S340, in step S350. The red light source control unit 110R repeats the processing of step S345 to step S350 until the detected light monitor value Qr becomes the target value Qr_B. Similarly, also the green light source control unit 110G and the blue light source control unit 110B repeat the increase or decrease of the drive currents and the determinations until the light monitor values Qg and Qb detected by the green light monitor unit 150G and the blue light monitor unit 150B become the target values Qg_B and Qb_B set in step S340 (step S355 to step S360 and step S365 to step S370).

The color temperature adjustment processing terminates when the light monitor values Qr, Qg, and Qb coincide with the target values Qr_B, Qg_B, and Qb_B for all of the red light source 130R, the green light source 130G, and the blue light source 130B, respectively. As a result, it is possible to adjust the color temperature of the illumination light to a color temperature set by a user or a preliminarily set color temperature, while maintaining the light amount of the illumination light. As thus described, the endoscopic system according to the present embodiment acquires the calibration formulas Fwr, Fwg, Fwb, Fr, Fg, and Fb expressing the correlations between the light monitor values and the luminances of the emission light beams from the white light source and the RGB light sources, after attachment of the endoscopic probe, for example. Accordingly, thereafter, it is possible to easily perform color temperature adjustment without referring to images acquired by the imaging processing device 200, while maintaining the light amount (Qx) of the illumination light.

Note that, in the flow of the color temperature adjustment processing illustrated in FIG. 17, although the luminance of the illumination light is corrected in accordance with the emission light beams emitted from the RGB light sources, mainly based on the white light emitted from the white light source 130W, the color temperature adjustment processing is not limited to such an example. The luminance of the illumination light may be corrected by the white light emitted from the RGB light sources mainly based on the emission light beams emitted from the white light source 130W.

### (3.2.3. Light amount adjustment processing example)

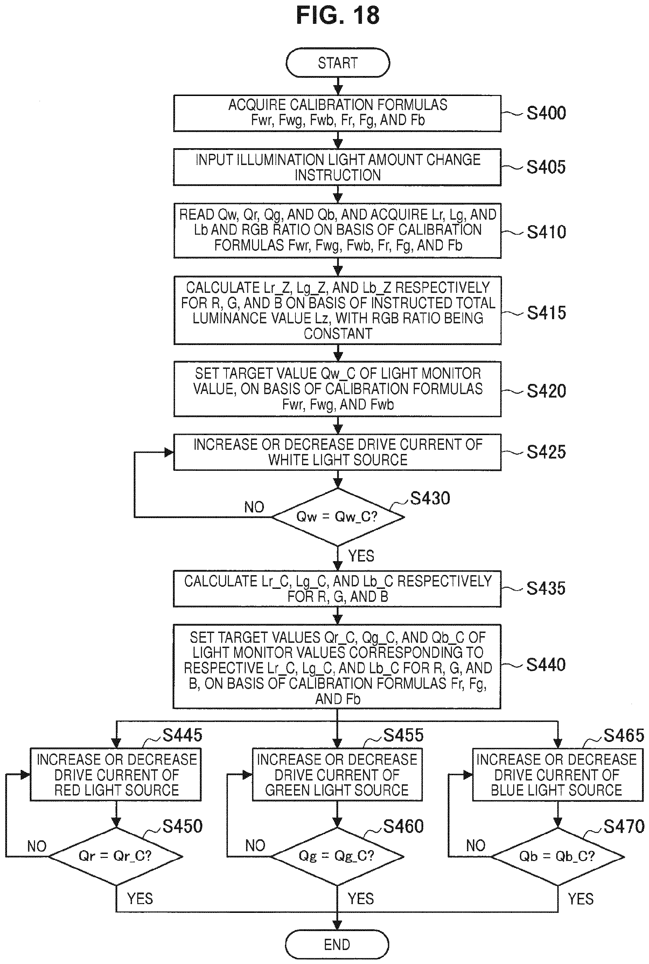

FIG. 18 illustrates a flowchart of a light amount adjustment processing example by the illuminating device 400 according to the present embodiment. This light amount adjustment processing flow is a processing flow to adjust the light amount of the illumination light while maintaining the color temperature of the currently radiated illumination light, that is, the luminance ratio of the red light, the green light, and the blue light (RGB ratio).

First, in step S400, for the white light, the white light source control unit 110W calculates the correlations between the light monitor value Qw of the emission light beams and the luminances Lwr, Lwg, and Lwb of the light having respective colors of R, G, and B. In addition, the red light source control unit 110R, the green light source control unit 110G, and the blue light source control unit 110B calculate the correlations between the light monitor values Qr, Qg, and Qb of the emission light beams, and the luminances Lr, Lg, and Lb, for the red light, the green light, and the blue light, respectively. This step S400, which is performed in a procedure illustrated in FIG. 13 similarly to step S600 of the white balance adjustment processing illustrated in FIG. 12, may be performed in the stage of attaching the endoscopic probe to the illuminating device 400.

Next, in step S405, the white light source control unit 110W, the red light source control unit 110R, the green light source control unit 110G, and the blue light source control unit 110B receive an input of an instruction to change the light amount of the illumination light. Such an instruction to change the light amount is input by a user, for example, when he/she adjusts a light amount adjustment dial or presses an un-illustrated switch having a light amount preliminarily set thereto. In addition, the instructed value of the light amount may be the luminance value of the illumination light detected by the light receiving unit 230, for example.

Next, in step S410, the control units of respective light sources read the light monitor values Qw, Qr, Qg, and Qb detected by the white light monitor unit 150W, the red light monitor unit 150R, the green light monitor unit 150G, and the blue light monitor unit 150B, respectively. The control units of respective light sources may exchange information of the light monitor values Qw, Qr, Qg, and Qb, or the control units of respective light sources may read all of the light monitor values Qw, Qr, Qg, and Qb, respectively. The respective control units then calculate the respective luminances Lr, Lg, and Lb of the light having respective colors of R, G, and B corresponding to the light monitor values Qw, Qr, Qg, and Qb, together with their luminance ratio (RGB ratio), on the basis of the previously calculated calibration formulas Fwr, Fwg, Fwb, Fr, Fg, and Fb.

For example, the luminance Lr of the red light in the illumination light is calculated as the sum of the luminance value acquired from the light monitor value Qr on the basis of the calibration formulas Fr, and the luminance value acquired from the light monitor value Qw on the basis of the calibration formula Fwr. The luminance Lg of the green light in the illumination light is also calculated on the basis of the calibration formulas Fg and Fwg. Similarly, the luminance Lb of the blue light in the illumination light is calculated on the basis of the calibration formulas Fb and Fwb. When the respective luminances Lr, Lg, and Lb are acquired, the RGB ratio of the illumination light is calculated. Calculation of the RGB ratio of the illumination light may be performed by any of the white light source control unit 110W, the red light source control unit 110R, the green light source control unit 110G, and the blue light source control unit 110B, and the RGB ratio may be output to other control units.

Next, in step S415, the red light source control unit 110R calculates a target luminance Lr_Z of the red light, at which the sum of the luminances of the red light, the green light, and the blue light becomes the luminance Lz corresponding to the instructed light amount of the illumination light, while maintaining the RGB ratio of the illumination light. Similarly, the green light source control unit 110G or the blue light source control unit 110B calculates target luminance Lg_Z, Lb_Z of the green light or the blue light, at which the sum of the luminances of the light having respective colors becomes the luminance value Lz corresponding to the instructed light amount of the illumination light, while maintaining the RGB ratio of the illumination light.

Next, in step S420, the white light source control unit 110W calculates the light monitor value Qw_B at which the luminances of the light having respective colors of R, G, and B all become equal to or lower than their respective target luminances Lr_Z, Lg_Z, and Lb_Z, on the basis of the calibration formulas Fwr, Fwg, and Fwb. The white light source control unit 110W then sets the calculated light monitor value Qw_C as the target value of drive control of the white light source 130W.

Next, in step S425, the white light source control unit 110W increases or decreases the drive current of the white light source 130W. Subsequently, in step S430, the white light source control unit 110W determines whether or not the light monitor value Qw detected by the white light monitor unit 150W has become the target value Qw_C set in step S420. The white light source control unit 110W repeats the processing of step S425 to step S430 until the detected light monitor value Qw becomes the target value Qw_C.

Next, in step S435, the red light source control unit 110R calculates a difference Lr_C between the target luminance Lr_Z of the red light in the illumination light and the luminance Lr of the red light corresponding to the light monitor value Qw_C of the current white light. In step S435, also for the green light source control unit 110G or the blue light source control unit 110B, difference Lg_C, Lb_C is similarly calculated between the target luminance Lg_Z, Lb_Z of the light having respective colors in the illumination light, and the luminance Lg, Lb of the light having respective colors corresponding to the light monitor value Qw_C of the current white light.

Next, in step S440, the red light source control unit 110R calculates the light monitor value Qr_C corresponding to the aforementioned difference Lr_C of the luminance, on the basis of the calibration formula Fr. The red light source control unit 110R then sets the calculated value Qr_C as the target value of drive control of the red light source 130R. In step S440, also the green light source control unit 110G or the blue light source control unit 110B similarly sets the light monitor value Qg_C, Qb_C to be the target value of drive control of the green light source 130G or the blue light source 130B, on the basis of the calibration formula Fg, Fb.

Next, the red light source control unit 110R increases or decreases the drive current of the red light source 130R in step S445. Subsequently, the red light source control unit 110R determines whether or not the light monitor value Qr detected by the red light monitor unit 150R has become the target value Qr_C set in step S440, in step S450. The red light source control unit 110R repeats the processing of step S345 to step S350 until the detected light monitor value Qr becomes the target value Qr_C. Similarly, also the green light source control unit 110G and the blue light source control unit 110B repeat the increase or decrease of the drive currents and the determinations until the light monitor values Qg and Qb detected by the green light monitor unit 150G and the blue light monitor unit 150B become the target values Qg_C and Qb_C set in step S440 (step S455 to step S460 and step S465 to step S470).

The light amount adjustment processing of illumination light terminates when the light monitor values Qr, Qg, and Qb coincide with the target values Qr_C, Qg_C, and Qb_C for all of the red light source 130R, the green light source 130G, and the blue light source 130B, respectively. As a result, it is possible to adjust the light amount of the illumination light to a light amount set by a user or a preliminarily set light amount, while maintaining the color temperature of the illumination light. As thus described, the endoscopic system according to the present embodiment acquires the calibration formulas Fwr, Fwg, Fwb, Fr, Fg, and Fb expressing the correlations between the light monitor values and the luminances of the emission light beams from the white light source and the RGB light sources, at the time of attachment of the endoscopic probe, for example. Accordingly, thereafter, it is possible to easily perform light amount adjustment without referring to images acquired by the imaging processing device 200, while maintaining the color temperature of the illumination light.

Note that, in the flow of the light amount adjustment processing illustrated in FIG. 18, although the luminance of the illumination light is corrected in accordance with the emission light beams emitted from the RGB light sources, mainly based on the white light emitted from the white light source 130W, the adjustment processing is not limited to such an example. The luminance of the illumination light may be corrected by the white light emitted from the RGB light sources mainly based on the emission light beams emitted from the white light source 130W.

### (3.2.4. Multiplexing ratio adjustment processing example)

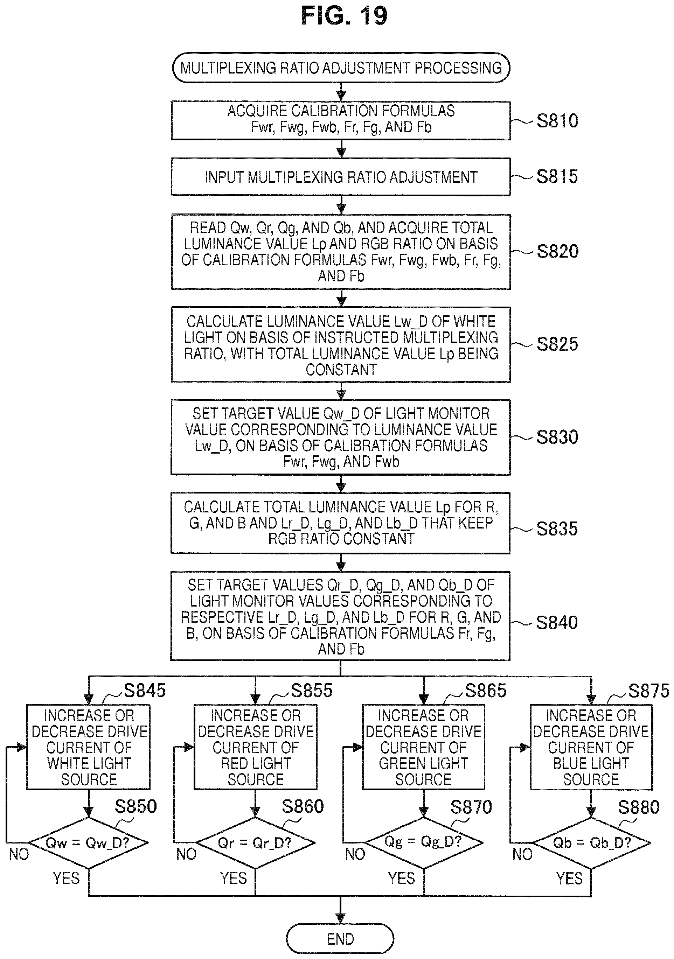

FIG. 19 illustrates a flowchart of a multiplexing ratio adjustment processing example performed by the illuminating device 400 according to the present embodiment. The flow of the multiplexing ratio adjustment processing is a flow of a process of adjusting the multiplexing ratio between the white light emitted from the white light source 130W and emission light beams (also referred to as "RGB light" in the following) emitted from the RGB light sources, while maintaining the light amount and the color temperature (RGB ratio) of the illumination light currently being radiated.

First, in step S810, for the white light, the white light source control unit 110W calculates the correlations between the light monitor value Qw of the emission light beams and the luminances Lwr, Lwg, and Lwb of the light having respective colors of R, G, and B. In addition, the red light source control unit 110R, the green light source control unit 110G, and the blue light source control unit 110B calculate the correlations between the light monitor values Qr, Qg, and Qb of the emission light beams, and the luminances Lr, Lg, and Lb, for the red light, the green light, and the blue light, respectively. This step S810, which is performed in a procedure illustrated in FIG. 13 similarly to step S600 of the white balance adjustment processing illustrated in FIG. 12, may be performed in the stage of attaching the endoscopic probe to the illuminating device 400.

Next, in step S815, the white light source control unit 110W, the red light source control unit 110R, the green light source control unit 110G, and the blue light source control unit 110B receive an input of an instruction to adjust the multiplexing ratio. Such an instruction to adjust the multiplexing ratio is input by a user, for example, when he/she operates a multiplexing ratio adjustment dial or presses an un-illustrated switch having a multiplexing ratio preliminarily set thereto. However, step S815 may be omitted in a case where the time of performing the multiplexing ratio adjustment processing, such as the start time of using the illuminating device 400, is preliminarily set.

Next, in step S410, the control units of respective light sources read the light monitor values Qw, Qr, Qg, and Qb detected by the white light monitor unit 150W, the red light monitor unit 150R, the green light monitor unit 150G, and the blue light monitor unit 150B, respectively. The control units of respective light sources may exchange information of the light monitor values Qw, Qr, Qg, and Qb, or the control units of respective light sources may read all of the light monitor values Qw, Qr, Qg, and Qb, respectively. The respective control units then calculate the respective luminances Lr, Lg, and Lb of the light having respective colors of R, G, and B corresponding to the light monitor values Qw, Qr, Qg, and Qb, together with their the total luminance value Lp and luminance ratio (RGB ratio), on the basis of the previously calculated calibration formulas Fwr, Fwg, Fwb, Fr, Fg, and Fb.

For example, the luminance Lr of the red light in the illumination light is calculated as the sum of the luminance value acquired from the light monitor value Qr on the basis of the calibration formulas Fr, and the luminance value acquired from the light monitor value Qw on the basis of the calibration formula Fwr. The luminance Lg of the green light in the illumination light is also calculated on the basis of the calibration formulas Fg and Fwg. Similarly, the luminance Lb of the blue light in the illumination light is calculated on the basis of the calibration formulas Fb and Fwb. A total luminance value Lp is calculated by addition of the respectively acquired luminances Lr, Lg, and Lb. In addition, when the respective luminances Lr, Lg, and Lb are acquired, the RGB ratio of the illumination light is calculated. Calculation of the total luminance value Lp and the RGB ratio of the illumination light may be performed by the control unit of any of the light sources, and the RGB ratio may be output to other control units.

Next, in step S825, the white light source control unit 110W calculates a total luminance value Lw_D of the white light emitted from the white light source 130W on the basis of the instructed multiplexing ratio, while keeping constant the total luminance value Lp of the illumination light. Specifically, when the instructed ratio between the white light and RGB light is S:T, for the total luminance value Lp of the illumination light, the total luminance value Lw_D of the white light becomes LpxS/(S+T).

Next, in step S830, the white light source control unit 110W calculates the light monitor value Qw_D at which the sum of the luminances Lwr, Lwg, and Lwb of the light having respective colors of R, G, and B becomes the total luminance value Lw_D, on the basis of the calibration formulas Fwr, Fwg, and Fwb. The white light source control unit 110W then sets the calculated light monitor value Qw_D as the target value of drive control of the white light source 130W.

Next, in step S835, the red light source control unit 110R, the green light source control unit 110G, and the blue light source control unit 110B calculate the luminances Lr_D, Lg_D, and Lb_D of the light having respective colors required to maintain the RGB ratio of the illumination light, respectively. On this occasion, the value of the sum of the luminances Lr_D, Lg_D, and Lb_D of respective colors becomes the value resulting from subtracting the luminance value Lw_D of the white light from the total luminance value Lp of the illumination light. For example, when the instructed ratio between the white light and the RGB light is S:T for the total luminance value Lp of the illumination light, the value of the sum of the luminances Lr_D, Lg_D, and Lb_D of respective colors becomes LpxT/ (S+T).

Next, in step S840, the red light source control unit 110R calculates the light monitor value Qr_D corresponding to the aforementioned difference Lr_D of the luminance, on the basis of the calibration formula Fr. The red light source control unit 110R then sets the calculated value Qr_D as the target value of drive control of the red light source 130R. In step S840, also the green light source control unit 110G or the blue light source control unit 110B similarly sets the light monitor value Qg_D, Qb_D to be the target value of drive control of the green light source 130G or the blue light source 130B, on the basis of the calibration formula Fg, Fb.

Next, the white light source control unit 110W increases or decreases the drive current of the white light source 130W in step S845. Subsequently, in step S850, the white light source control unit 110W determines whether or not the light monitor value Qw detected by the white light monitor unit 150W has become the target value Qw_D set in step S830. The white light source control unit 110W repeats the processing of step S845 to step S850 until the detected light monitor value Qw becomes the target value Qr_D.

Next, the red light source control unit 110R increases or decreases the drive current of the red light source 130R in step S855. Subsequently, the red light source control unit 110R determines whether or not the light monitor value Qr detected by the red light monitor unit 150R has become the target value Qr_D set in step S840, in step S860. The red light source control unit 110R repeats the processing of step S8555 to step S860 until the detected light monitor value Qr becomes the target value Qr_D. Similarly, also the green light source control unit 110G and the blue light source control unit 110B repeat the increase or decrease of the drive currents and the determinations until the light monitor values Qg and Qb detected by the green light monitor unit 150G and the blue light monitor unit 150B become the target values Qg_D and Qb_D set in step S840 (step S865 to step S870 and step S875 to step S880).

The multiplexing ratio adjustment processing of the illumination light terminates when the light monitor values Qw, Qr, Qg, and Qb coincide with the target values Qw_D, Qr_D, Qg_D, and Qb_D for all of the white light, the red light, the green light and the blue light, respectively. As a result, it is possible to adjust the multiplexing ratio of the illumination light to a ratio set by a user or a preliminarily set ratio, while maintaining the light amount and the color temperature of the illumination light. Since the illuminating device 400 according to the present embodiment can adjust the multiplexing ratio between the white light and the RGB light, it is possible to mutually supplement the disadvantages of the white light source and the RGB light sources.

For example, the white light source 130W has a large etendue (light-emitting area of light source x solid angle of light diffused from light source) and therefore the coupling efficiency tends to decrease in an endoscopic system with a small probe inner diameter, thereby resulting in a dark illumination. In contrast, laser-based RGB light sources, in which the coupling efficiency does not decrease, can also support an endoscopic probe with a small inner diameter, thereby supplementing the brightness of illumination. In laser-based RGB light sources, on the other hand, there may occur speckle noise intrinsic to laser beam due to interference between coherent light beams in a random phase relationship. In contrast, the white light emitted from the white light source is incoherent light, and speckle noise is suppressed by mixing of the white light in the RGB light.

As thus described, the endoscopic system according to the present embodiment acquires the calibration formulas Fwr, Fwg, Fwb, Fr, Fg, and Fb expressing the correlations between the light monitor values and the luminance values of the emission light beams from the white light source and the RGB light sources at the time of attachment of the endoscopic probe, for example. Accordingly, thereafter, it is possible to easily adjust the multiplexing ratio without referring to images acquired by the imaging processing device 200, while maintaining the light amount and the color temperature of the illumination light.

Note that, in the flow of multiplexing adjustment processing illustrated in FIG. 19, although the target values Qr_D, Qg_D, and Qb_D of the light monitor value of the RGB light sources are calculated after acquisition of the target value Qw_D of the light monitor value of the white light source 130W, the order of calculating the target values is not limited to such an example.

In the illuminating device 400 and the image acquisition system 30 according to the third embodiment of the present disclosure as has been described above, the light amounts of the light emitted from the white light source 130W, the red light source 130R, the green light source 130G, and the blue light source 130B are respectively detected by the light monitor unit. In addition, the illuminating device 400 and the image acquisition system 30 described above acquire the calibration formulas Fwr, Fwg, Fwb, Fr, Fg, and Fb expressing the correlations between the luminances detected by the light receiving unit 230 and the light monitor values for respective light sources. Accordingly, thereafter, it is possible to accurately adjust the light amount and the color temperature of the illumination light by adjusting the light amounts of respective light sources on the basis of the calibration formulas Fwr, Fwg, Fwb, Fr, Fg, and Fb without taking into account the influence of the endoscopic probe, as long as the same endoscopic probe is kept using.

In addition, the illuminating device 400 and the image acquisition system 30 according to the present embodiment once calculate the calibration formulas Fwr, Fwg, Fwb, Fr, Fg, and Fb expressing the correlations between respective light monitor values and luminances detected by the light receiving unit 230 at the time of attachment of the endoscopic probe, for example. Accordingly, even in a case where different endoscopic probes are used, it is possible to accurately adjust the light amount and the color temperature of the illumination light by calculating the calibration formulas Fr, Fg, and Fb at the time of attachment of the respective endoscopic probes. Therefore, a desired captured image suitable for the imaging object can be acquired regardless of individual differences among endoscopic probes in an endoscopic system used in the medical front, for example.

In particular, the illuminating device 400 and the image acquisition system 30 according to the present embodiment can also control the light amount of the white light in the white light source 130W to a light amount of a desired luminance, on the basis of the calibration formulas Fwr, Fwg, and Fwb. Accordingly, it is possible to accurately adjust the white balance, the color temperature, the light amount, and also the multiplexing ratio of the illumination light, without referring to images acquired by the imaging processing device 200 each time. The light amount and the color temperature required for the illumination light vary depending on the user and the purpose of use. In addition, it is also conceivable that, depending on the user, the illumination light is desired to have a high ratio of white light emitted from the white light source 130W, or a high ratio of emission light beams emitted from the RGB light sources. The illuminating device 400 and the image acquisition system 30 according to the present embodiment allow for a higher degree of freedom of adjusting the light amount, the color temperature, the multiplexing ratio, or the like of the radiated illumination light.

Note that, although the white light source 130W, the red light source 130R, the green light source 130G, and the blue light source 130B are respectively controlled by separate control units in the illuminating device 400 according to the present embodiment, all of the light sources may be controlled by a common control unit as illustrated in FIG. 9.

### <4. Fourth embodiment>

A fourth embodiment of the present disclosure differs from the respective embodiments described above in that the control units of respective light sources have a function as a deterioration determination unit configured to determine deterioration of a light source. For example, the red light source control unit 110R, the green light source control unit 110G, and the blue light source control unit 110B according to the first embodiment, and the control unit 330 according to the second embodiment may have the function as the deterioration determination unit configured to determine deterioration of the light source. In addition, the white light source control unit 110W, the red light source control unit 110R, the green light source control unit 110G, and the blue light source control unit 110B according to the third embodiment may have the function as the deterioration determination unit configured to determine deterioration of the light source.

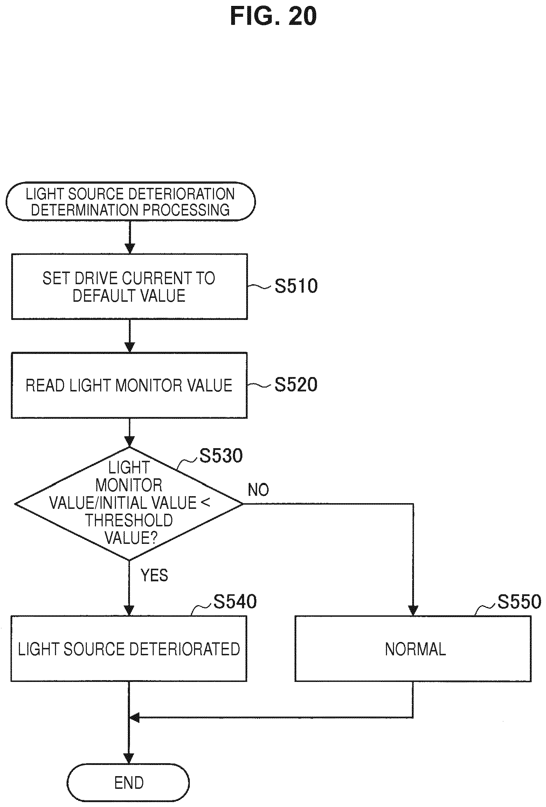

An illuminating device and an image acquisition system according to the present embodiment can be configured basically the same as the illuminating devices 100, 300, and 400 and the image acquisition systems 10, 20, and 30 illustrated in FIG. 1, FIG. 9, and FIG. 10, respectively. On the other side, each of the illuminating device and the image acquisition system according to the present embodiment has a function of determining the deterioration of the light source in which the light amount is reduced along the elapse of the use time even when the same current is supplied. In the following, the illuminating device and the image acquisition system according to the present embodiment will be explained with reference to FIG. 20 using the illuminating device 400 and the image acquisition system 30 illustrated in FIG. 10 as an example.

FIG. 20 is a flowchart illustrating a deterioration determination processing example of the light source. In the case of the illuminating device 400 illustrated in FIG. 10, the deterioration determination processing of each light source is carried out by the corresponding control unit. For example, explaining the deterioration determination of the red light source 130R, first in step S510, the red light source control unit 110R sets the drive current supplied to the red light source 130R to a default value. Such a default may be set arbitrarily to a value which can detect the change of the light amount of the emission light beams emitted from the red light source 130R. Such a default is used as a common value when the deterioration determination of the red light source 130R is carried out. The same value may be set as the default for each of the light sources or the default may be different among the light sources.

Next, in step S520, the red light source control unit 110R reads the light monitor value Qr detected by the red light monitor unit 150R in the state that the drive current having the default is supplied to the red light source 130R. Subsequently, in step S530, the red light source control unit 110R determines whether or not the ratio R of the current light monitor value Qr to the initial value Qr0 of the light monitor value detected when the current having the same default is supplied to the red light source 130R at the use start is smaller than a preliminarily determined threshold value R0. If the ratio R is not smaller than the threshold value R0 (No in S530), the process proceeds to step S550, and the red light source control unit 110R determines that there is no deterioration in the red light source 130R and the process is finished. On the other side, if the ratio R is smaller than the threshold value R0 (Yes in S530), the process proceeds to step S540, and the red light source control unit 110R determines that the red light source 130R is deteriorated and the process is finished.

In the case of the illuminating device 400 illustrated in FIG. 10, also the white light source control unit 110W, the green light source control unit 110G and the blue light source control unit 110B can carry out the deterioration determination processing of the white light source 130W, the green light source 130G and the blue light source 130B, respectively, in similar sequences. Further, in the case of the illuminating device 300 illustrated in FIG. 9, the control unit 330 can carry out the deterioration determination of each of the light sources sequentially along the sequence illustrated in FIG. 20.

Each of the above explained illuminating devices and the image acquisition systems according to the present embodiments includes the light monitor units 150W, 150 R, 150G, and 150B to detect the light amounts of the emission light beams emitted from the light sources, respectively Accordingly, the control units can determine the deterioration of the light sources on the basis of the reduction ratios of the light monitor values Qw, Qr, Qg, and Qb in the state that the same drive currents are supplied to the light sources, respectively.

### <5. Fifth embodiment>

A fifth embodiment of the present disclosure differs from the respective embodiments described above in that a single light monitor unit detects the light amounts of emission light beams respectively emitted from a plurality of light sources. In the following, description will be provided mainly on different points from the illuminating device according to the respective embodiments.

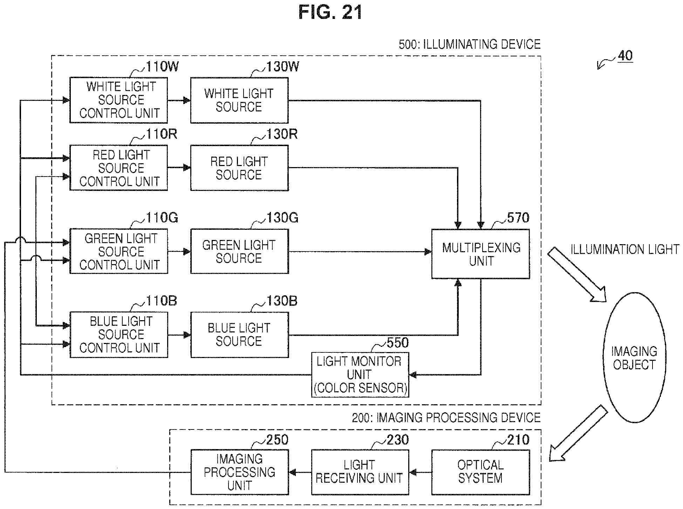

FIG. 21 is a block diagram illustrating an overall configuration of an image acquisition system 40 according to the present embodiment. The image acquisition system 40 includes an illuminating device 500 and an imaging processing device 200, and is configured as an endoscopic system, for example. Among these, the imaging processing device 200 may be configured similarly to the imaging processing device 200 of the image acquisition system according to the respective embodiments described above.

The illuminating device 500 includes the white light source 130W, the red light source 130R, the green light source 130G, the blue light source 130B, the white light source control unit 110W, the red light source control unit 110R, the green light source control unit 110G, the blue light source control unit 110B and a multiplexing unit 570. In addition, the illuminating device 500 includes a color sensor 550 as a single light monitor unit. The white light source 130W, the red light source 130R, the green light source 130G, and the blue light source 130B can be configured similarly to the light sources of the illuminating device 100 according to the first embodiment. In addition, although the light amount of the white light source 130W can also be controlled in the illuminating device 500 according to the present embodiment, the light amount of the white light source 130W may be kept constant as with the illuminating device 500 according to the first embodiment. In addition, the light source is not limited to the light sources for three colors R, G, and B but may be light sources for four colors or the like, and the number of light sources is not limited.

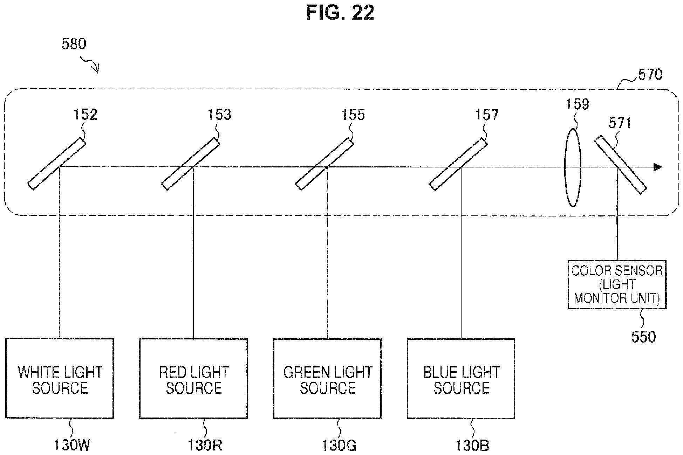

The multiplexing unit 570 multiplexes white light with light having respective colors of R, G, and B, and emits the multiplexed light as illumination light. FIG. 22 is a schematic diagram illustrating a configuration example of a multiplexing module 580 with the color sensor 550, including the multiplexing unit 570. The multiplexing unit 570 of the illuminating device 500 according to the present embodiment includes a dichroic mirror 571 configured to reflect a part of the light multiplexed and collected by the lens 159, and change the course of the reflected light toward the color sensor 550. In the multiplexing module 180 described above, the multiplexed light is made to enter the color sensor 550 so that the color sensor 550 detects the light amount of individual light beam, instead of detecting the light amount of light emitted from respective light sources by separate light monitor units.

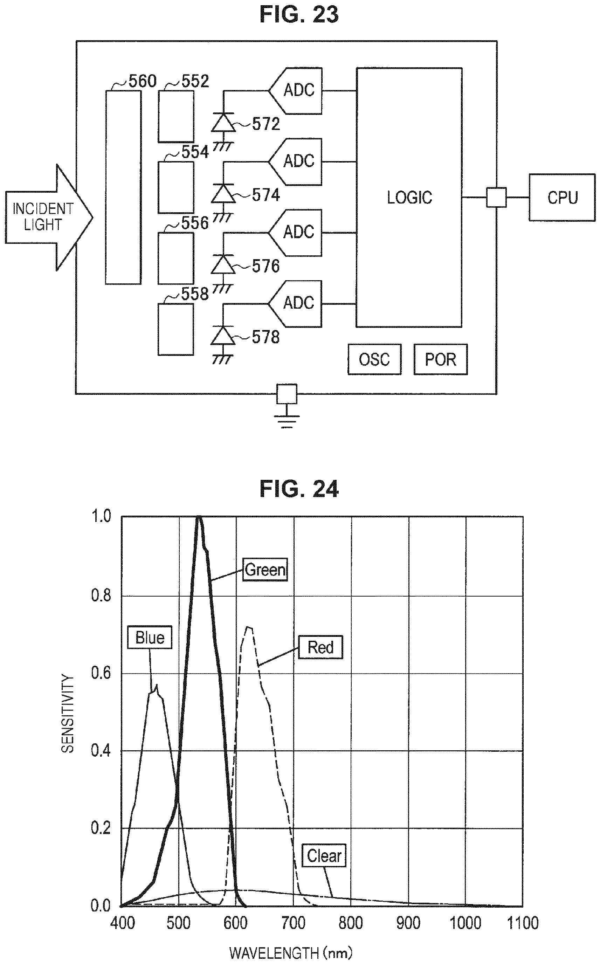

FIG.23 is a schematic diagram illustrating a configuration example of the color sensor 550. The color sensor 550 includes an infrared cut filter 560, a red light filter 552, a green light filter 554, a blue light filter 556, a clear light transmission unit 558, and light detecting units 572 to 578 for detecting the light amounts of the light having respective colors. Light which has passed through the infrared cut filter 560 further transmits through the red light filter 552, the green light filter 554, or the blue light filter 556, respectively. The light detecting unit 572 detects the light amount of the light which has passed through the red light filter 552, the light detecting unit 574 detects the light amount of the light which has passed through green light filter 554. In addition, the light detecting unit 576 detects the light amount of the light which has passed through the blue light filter 556, and the light detecting unit 578 detects the light amount of clear light which does not transmit through the filter.

FIG. 24 illustrates an example of spectral sensitivity property of the color sensor 550. The color sensor 550 can cut light having a wavelength in the infrared region and also disperse the light into light of respective wavelengths of the red light, the green light, and the blue light so as to detect the light amount of the light having respective colors, by causing the incident light to transmit through respective filters. In addition, the color sensor 550 of the configuration illustrated in FIG. 23 can also detect the light amount of the clear light which does not pass through the filter.

The light amount detected by the light detecting unit 572 is converted into a voltage signal as the light amount of the red light and transmitted to the red light source control unit 110R. The light amount detected by the light detecting unit 574 is converted into a voltage signal as the light amount of green light and transmitted to the green light source control unit 110G. The light amount detected by the light detecting unit 576 is converted into a voltage signal as the light amount of the blue light and transmitted to the blue light source control unit 110B. The light amount detected by the light detecting unit 578 is converted into a voltage signal as the light amount of the white light and transmitted to the white light source control unit 110W.

The red light source control unit 110R, the green light source control unit 110G, and the blue light source control unit 110B can be basically configured similarly to the control units of the respective light sources of the illuminating device 100 according to the first embodiment. In other words, the control unit of each of the RGB light sources calculates the correlation (calibration formula) between the luminance Lr (Lg, Lb) detected by the light receiving unit 230 of the imaging processing device 200 and the light monitor value Qr (Qg, Qb) detected by the color sensor 550. In addition, the control unit of each of the RGB light sources controls the drive current of each light source on the basis of the calculated correlation.

In addition, the white light source control unit 110W can be basically configured similarly to the white light source control unit 110W of the illuminating device 400 according to the third embodiment. That is, the white light source control unit 110W controls the drive current supplied to the white light source 130W, on the basis of the correlations between the luminances Lwr, Lwg, and Lwb detected by the light receiving unit 230 of the imaging processing device 200, and the light monitor value Qw detected by the color sensor 550. The white light source control unit 110W turns on only the white light source 130W with a different light amount, and calculates the correlations between the luminances Lwr, Lwg, and Lwb of the light having respective colors of R, G, and B detected by the light receiving unit 230 on that occasion, and the light monitor value Qw of the white light detected by the white light monitor unit 150W. In other words, the white light source control unit 110W calculates three correlations corresponding to respective colors of R, G, and B. Note that the light receiving unit 230 is capable of dispersing the received white light into light having respective colors of R, G, and B, and detecting the luminances Lwr, Lwg, and Lwb of the light having respective colors.

The illuminating device 500 according to the present embodiment can detect the light amount of the emission light beams emitted from respective light sources by a single color sensor (light monitor unit) 550, and perform each control processing illustrated in the illuminating device according to the respective embodiments described above. Therefore, the illuminating device 500 and the image acquisition system 40 according to the present embodiment can provide the same effect as the illuminating device and the image acquisition system according to the respective embodiments described above.

The preferred embodiment(s) of the present disclosure has/have been described above with reference to the accompanying drawings, whilst the present disclosure is not limited to the above examples. A person skilled in the art may find various alterations and modifications within the scope of the appended claims, and it should be understood that they will naturally come under the technical scope of the present disclosure.

For example, although it is assumed in the aforementioned embodiment that the luminances Lr_X, Lg_X, and Lb_X of the light having respective colors of R, G, and B in the illumination light are preliminarily set in the white balance adjustment processing, the present technique is not limited to such an example. For example, the luminances Lr_X, Lg_X, and Lb_X may be arbitrarily set by the user at the time of white balance adjustment processing.

Further, while, in the above embodiments, each of the white light source control unit, the red light source control unit, the green light source control unit, and the blue light source control unit or the control unit calculates the calibration formula, the present technique is not limited to such an example. For example, a calculation processing unit to calculate the calibration formula may be a device separate from each of the control units.

Further, while, in the above embodiments, each of the control units 110W, 110R, 110G, 110B, or 330 increases or decreases the drive current after the target light monitor value is acquired, the present technique is not limited to such an example. For example, after the target light monitor value is acquired, a user may adjust the drive current in each of the light sources.

Further, while, in the above embodiments, when the color temperature adjustment processing and the light amount adjustment processing are carried out, the color temperature is adjusted while the light amount of the illumination light is maintained, or the light amount is adjusted while the color temperature of the illumination light is maintained, the present technique is not limited to such an example. For example, the light amount in each of the light sources can be adjusted so as to cause both of the color temperature (RGB ratio) and the light amount of the illumination light to become respective set target values. In this case, a luminance value in each of the color light beams may be acquired on the basis of a total luminance value corresponding to the set light amount and the set RGB ratio, and a light monitor value corresponding to the luminance value may be set to the target value. Thereby, it is possible to adjust the light amount and the color temperature of the illumination light by adjusting the light amount in each of the color light beams.

Further, the effects described in this specification are merely illustrative or exemplified effects, and are not limitative. That is, with or in the place of the above effects, the technology according to the present disclosure may achieve other effects that are clear to those skilled in the art from the description of this specification.

Additionally, the present technology may also be configured as below.
(1) An illuminating device including:
   a white light source configured to emit white light;
   RGB light sources light amounts of respective emission light beams of which are independently adjustable;
   a multiplexing unit configured to multiplex the white light and the emission light beams into illumination light; and
   a control unit configured to control the light amounts of the respective emission light beams.
(2) The illuminating device according to (1), including:
   a light monitor unit configured to detect the light amounts of the respective emission light beams emitted from the RGB light sources.
(3) The illuminating device according to (2), further including:
   a light monitor unit configured to detect a light amount of the white light emitted from the white light source.
(4) The illuminating device according to (2), wherein the control unit controls the light amounts of the respective emission light beams, on a basis of correlations between the light amounts of the emission light beams and a luminance acquired from a light receiving unit.
(5) The illuminating device according to (3), wherein the control unit controls respective light amounts of the white light and the emission light beams, on a basis of correlations between the light amounts of the white light and the emission light beams, and a luminance acquired from a light receiving unit.
(6) The illuminating device according to (4), wherein the control unit calculates the correlations, for the respective emission light beams, on a basis of luminances acquired when the emission light beams are emitted with different light amounts.
(7) The illuminating device according to (5), wherein the control unit calculates the correlations between the light amount of the white light and luminances having respective colors of R, G, and B acquired when the white light is emitted with different light amounts, on a basis of the luminances having the respective colors of R, G, and B.
(8) The illuminating device according to any one of (1) to (7), wherein the white light source is an LED light source, and the RGB light sources are laser light sources.
(9) The illuminating device according to any one of (2) to (8), including:
   a deterioration determination unit configured to determine deterioration of the RGB light sources, on a basis of light amounts detected by the light monitor unit.
(10) A control method for an illuminating device, the control method including:
   controlling light amounts of respective emission light beams emitted from RGB light sources so as to acquire illumination light of a desired luminance, the respective emission light beams being multiplexed with white light emitted from a white light source.
(11) The control method for an illuminating device according to (10), the control method including:
   controlling the light amounts of the respective emission light beams, on a basis of correlations between the light amounts of the emission light beams and a luminance acquired from a light receiving unit.
(12) The control method for an illuminating device according to (11), the control method including:
   controlling the light amount of the white light, on a basis of correlations between the light amount of the white light and luminances having respective colors of R, G, and B acquired from the light receiving unit.
(13) The control method for an illuminating device according to any one of (10) to (12), the control method including:
   controlling the light amounts of the respective emission light beams, on a basis of correlations between the light amounts of the respective emission light beams and a luminance acquired from a light receiving unit, so that luminances having respective colors of R, G, and B in the illumination light become desired luminances.
(14) The control method for an illuminating device according to any one of (10) to (12), the control method including:
   controlling the light amounts of the respective emission light beams, on a basis of correlations between the light amounts of the respective emission light beams and a luminance acquired from a light receiving unit, so that luminances having respective colors of R, G, and B in the illumination light become identical.
(15) The control method for an illuminating device according to any one of (10) to (14), the control method including:
   controlling the light amounts of the respective emission light beams, on a basis of correlations between the light amounts of the respective emission light beams and a luminance acquired from a light receiving unit, so that a light amount of the illumination light is changed while maintaining a ratio of luminances having respective colors of R, G, and B in the illumination light.
(16) The control method for an illuminating device according to any one of (10) to (15), the control method including:
   controlling the light amounts of the respective emission light beams, on a basis of correlations between the light amounts of the respective emission light beams and a luminance acquired from a light receiving unit, so that a ratio of luminances having respective colors of R, G, and B in the illumination light is changed while maintaining a light amount of the illumination light.
(17) The control method for an illuminating device according to any one of (10) to (16), the control method including:
   controlling the light amounts of the respective emission light beams after having controlled the light amount of the white light so as not to exceed luminance values having respective colors of R, G, and B in the illumination light of the desired luminance, when controlling the light amounts of the respective emission light beams.
(18) An image acquisition system including:
   a white light source configured to emit white light;
   RGB light sources light amounts of respective emission light beams of which are independently adjustable;
   a multiplexing unit configured to multiplex the white light and the emission light beams into illumination light;
   a light receiving unit configured to receive the illumination light; and
   a control unit configured to control light amounts of the respective emission light beams.
(19) The image acquisition system according to (18), including:
   a calculation unit configured to calculate correlations between the light amounts of the respective emission light beams and a luminance detected by the light receiving unit.
(20) The image acquisition system according to (18) or (19), wherein the image acquisition system is an endoscopic system or an electronic microscope system.

### Reference Signs List

10, 20, 30, 40 image acquisition system
100, 300, 400, 500 illuminating device
110W white light source control unit
110R red light source control unit
110G green light source control unit
110B blue light source control unit
130W white light source
130R red light source
130G green light source
130B blue light source
131 excitation light source
133, 135 condensing lens
137 optical crystal
139 resonator mirror
141 wavelength conversion element
143 reflection unit
150W white light monitor unit
150R red light monitor unit
150G green light monitor unit
150B blue light monitor unit
151W, 151R, 151G, 151B light sampler
152 mirror
153, 155, 157, 571 dichroic mirror
159 lens
170 multiplexing unit
180 multiplexing module
200 imaging processing device
210 optical system
230 light receiving unit
250 imaging processing unit
300 illuminating device
310R red light source drive circuit
310G green light source drive circuit
310B blue light source drive circuit
330 control unit
550 color sensors (light monitor unit)

## Claims

1. An illuminating device comprising:
a white light source configured to emit white light;
RGB light sources light amounts of respective emission light beams of which are independently adjustable;
a multiplexing unit configured to multiplex the white light and the emission light beams into illumination light; and
a control unit configured to control the light amounts of the respective emission light beams.

2. The illuminating device according to claim 1, comprising:
a light monitor unit configured to detect the light amounts of the respective emission light beams emitted from the RGB light sources.

3. The illuminating device according to claim 2, further comprising:
a light monitor unit configured to detect a light amount of the white light emitted from the white light source.

4. The illuminating device according to claim 2, wherein the control unit controls the light amounts of the respective emission light beams, on a basis of correlations between the light amounts of the emission light beams and a luminance acquired from a light receiving unit.

5. The illuminating device according to claim 3, wherein the control unit controls respective light amounts of the white light and the emission light beams, on a basis of correlations between the light amounts of the white light and the emission light beams, and a luminance acquired from a light receiving unit.

6. The illuminating device according to claim 4, wherein the control unit calculates the correlations, for the respective emission light beams, on a basis of luminances acquired when the emission light beams are emitted with different light amounts.

7. The illuminating device according to claim 5, wherein the control unit calculates the correlations between the light amount of the white light and luminances having respective colors of R, G, and B acquired when the white light is emitted with different light amounts, on a basis of the luminances having the respective colors of R, G, and B.

8. The illuminating device according to claim 1, wherein the white light source is an LED light source, and the RGB light sources are laser light sources.

9. The illuminating device according to claim 2, comprising:
a deterioration determination unit configured to determine deterioration of the RGB light sources, on a basis of light amounts detected by the light monitor unit.

10. A control method for an illuminating device, the control method comprising:
controlling light amounts of respective emission light beams emitted from RGB light sources so as to acquire illumination light of a desired luminance, the respective emission light beams being multiplexed with white light emitted from a white light source.

11. The control method for an illuminating device according to claim 10, the control method comprising:
controlling the light amounts of the respective emission light beams, on a basis of correlations between the light amounts of the emission light beams and a luminance acquired from a light receiving unit.

12. The control method for an illuminating device according to claim 11, the control method comprising:
controlling the light amount of the white light, on a basis of correlations between the light amount of the white light and luminances having respective colors of R, G, and B acquired from the light receiving unit.

13. The control method for an illuminating device according to claim 10, the control method comprising:
controlling the light amounts of the respective emission light beams, on a basis of correlations between the light amounts of the respective emission light beams and a luminance acquired from a light receiving unit, so that luminances having respective colors of R, G, and B in the illumination light become desired luminances.

14. The control method for an illuminating device according to claim 10, the control method comprising:
controlling the light amounts of the respective emission light beams, on a basis of correlations between the light amounts of the respective emission light beams and a luminance acquired from a light receiving unit, so that luminances having respective colors of R, G, and B in the illumination light become identical.

15. The control method for an illuminating device according to claim 10, the control method comprising:
controlling the light amounts of the respective emission light beams, on a basis of correlations between the light amounts of the respective emission light beams and a luminance acquired from a light receiving unit, so that a light amount of the illumination light is changed while maintaining a ratio of luminances having respective colors of R, G, and B in the illumination light.

16. The control method for an illuminating device according to claim 10, the control method comprising:
controlling the light amounts of the respective emission light beams, on a basis of correlations between the light amounts of the respective emission light beams and a luminance acquired from a light receiving unit, so that a ratio of luminances having respective colors of R, G, and B in the illumination light is changed while maintaining a light amount of the illumination light.

17. The control method for an illuminating device according to claim 10, the control method comprising:
controlling the light amounts of the respective emission light beams after having controlled the light amount of the white light so as not to exceed luminance values having respective colors of R, G, and B in the illumination light of the desired luminance, when controlling the light amounts of the respective emission light beams.

18. An image acquisition system comprising:
a white light source configured to emit white light;
RGB light sources light amounts of respective emission light beams of which are independently adjustable;
a multiplexing unit configured to multiplex the white light and the emission light beams into illumination light;
a light receiving unit configured to receive the illumination light; and
a control unit configured to control light amounts of the respective emission light beams.

19. The image acquisition system according to claim 18, comprising:
a calculation unit configured to calculate correlations between the light amounts of the respective emission light beams and a luminance detected by the light receiving unit.

20. The image acquisition system according to claim 18, wherein the image acquisition system is an endoscopic system or an electronic microscope system.
